# EUROPEAN PATENT SPECIFICATION

(11) **EP 0 372 019 B1**
(45) Date of publication and mention of the grant of the patent: **31.05.1995**
(21) Application number: 89902720.5
(22) Date of filing: 10.02.1989
(51) Int. Cl.: C12N 15/00, C12P 19/34, C07H 21/04

(54) **RHOPTRY MEMBRANE ANTIGEN OF $i(PLASMODIUM FALCIPARUM)**
RHOPTRY-MEMBRAN-ANTIGEN VON $i(PLASMODIUM FALCIPARUM)
ANTIGENE DE MEMBRANE DE RHOPTRIES DU $i(PLASMODIUM FALCIPARUM)

(30) Priority: 12.02.1988 AU 6743/88
(43) Date of publication of application: 13.06.1990
(73) Proprietor: SARAMANE PTY. LTD., Kooyong, VIC 3144 (AU)
(72) Inventor: PETERSON, Michael, Gregory, Lower Templestowe, VIC 3107 (AU); CREWTHER, Pauline, Elizabeth, North Carlton, VIC 3054 (AU); SMYTHE, Jason, Arthur, Edithvale, VIC 3196 (AU); MARSHALL, Vikki, Maree, Endeavour Hills, VIC 3801 (AU); SILVA, Anabel, West Footscray, VIC 3012 (AU); ANDERS, Robin, Fredric, North Melbourne, VIC 3051 (AU); KEMP, David, James, North Balwyn, VIC 3103 (AU); CULVENOR, Janetta, Gladys, Greensborough, VIC 3088 (AU); EDWARDS, Stirling, John, Northcote, VIC 3070 (AU); PYE, David, Bullengarook, VIC 3437 (AU)
(74) Representative: Holmes, Michael John
(86) International application number: AU8900056
(87) International publication number: WO8907645

(56) References cited:
- WO-A-84/02917
- PARASITOL RESEARCH, vol. 73, 1987, pages 425-434; J.T. CLARK et al.:"Identification and characterisation of proteins associated with the rhoptryorganelles of plasmodium falciparum merozoites"
- PROC. NATL. ACAD. SCI. USA, vol. 81, April 1984, pages 2456-2460; H.D. STAHL et al.:
- CHEMICAL ABSTRACTS, vol. 108, no. 21, 23rd May 1988, page 525, abstract no.184761b, Columbus, Ohio, US; G.R. BUSHELL et al.: "An antigenic in therhoptries of plasmodium falciparum", & MOL. BIOCHEM.PARASITOL. 1988, 28(2), 105-12
- Molecular and Biochemical Parasitology, 18: 183-195 (1986) Schofield L., et al.
- Am. J. Trop. Med. Hyg. 33(6): 1051-1054 (1984) Campbell G. et al.
- Am. J. Trop. Med. Hyg. (33)6: 1055-1059 (1984) Howard R. et al.
- R. F. Anders, In: The Biology Parasitism, Published 1988 by Alan R. Lisj, New York, Englund P. and Sher A. (Eds.) pp. 201-224

## Description

This invention relates to the identification of an antigen of the asexual blood stages of Plasmodium falciparum, which is potentially capable of generating an immune response and antibodies which are able to inhibit the growth of the parasite, and to the use of this antigen and antibodies to it in immunization, diagnostic and treatment methods.

The molecular events which occur when a malaria merozoite invades the host erythrocyte are poorly understood. Parasite molecules contained within the rhoptries, flask-shaped secretory organelles at the apical end of the merozoite, are believed to play a critical role in invasion. In the electron microscope the rhoptries appear as membrane-bound electron-dense structures. The invasion process coincides with the depletion of rhoptry contents which presumably are discharged via the apical duct although Bannister et.al. (1986) has suggested that discharge may also occur at the time of schizont rupture. Electronmicroscopy using tannic acid-containing fixatives has revealed multilamellar membraneous whorls derived from rhoptry secretions. This indicates that there is lipid in the rhoptry secretion and it has been suggested that this may contribute to the formation of the parasitophorous vacuole membrane.

A number of different proteins have been identified in the rhoptries of P.falciparum. Two antigen complexes have been identified. A high molecular weight complex is composed of three antigenic polypeptides of -M, 105,000, M, 135,000 and M, 145,000 (Campbell et.al. 1984; Holden et.al. 1985; Cooper et.al. 1988 and Lustigman et.al. 1988) and a low molecular weight complex is composed of several polypeptides derived from a M, 83,000 protein and a doublet of approximate M, 40,000 (Campbell et.al, 1984; Howard et.al., 1985; Braun-Breton et.al., 1986; Schofield et.al. 1986; Bushell et.al. 1988).

Rhoptry antigens are considered potential vaccine components, initially because a rhoptry antigen from P.yoelii (M, 235,000) effectively immunized mice against infection (Holder and Freeman, 1981). Subsequently, some monoclonal antibodies that inhibited the growth of P.falciparum in vitro were shown to be directed against rhoptry components (Schofield et.al., 1986). Furthermore, monkeys immunized with purified rhoptry antigens were partially protected against challenge with P.falciparum (Perrin et.al., 1985; Siddiqui et.al., 1987).

According to one aspect of the present invention, there is provided a rhoptry membrane antigen of the asexual blood stages of Plasmodium falciparum, which is characterised by:
(i) being initially synthesized as a polypeptide having a relative molecular mass of approximately 80,000;
(ii) being located in the neck region (peduncle) of the rhoptries of merozoites; and
(iii) having the solubility characteristics and a hydrophobic domain typical of an integral membrane protein;

or an antigenic fragment thereof.

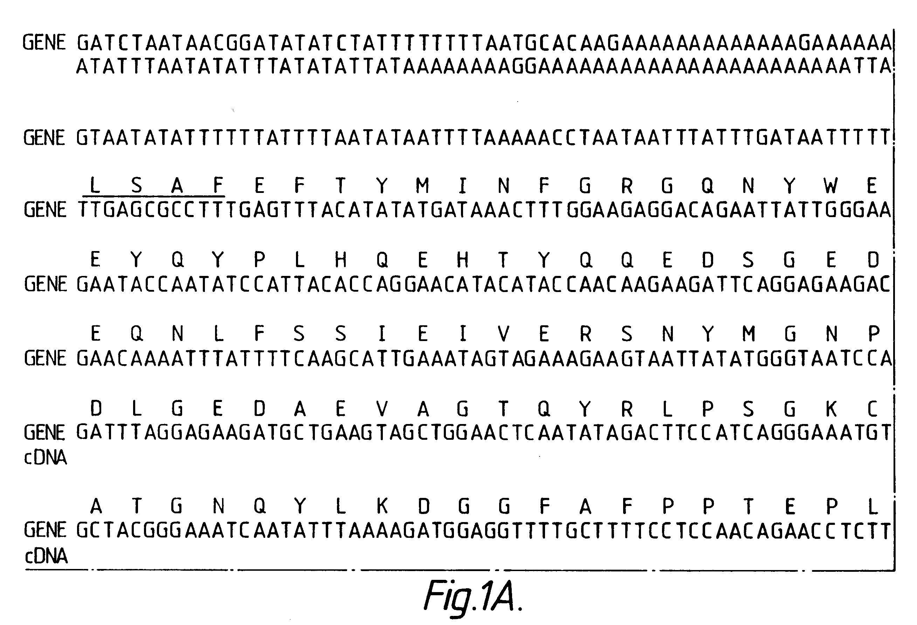

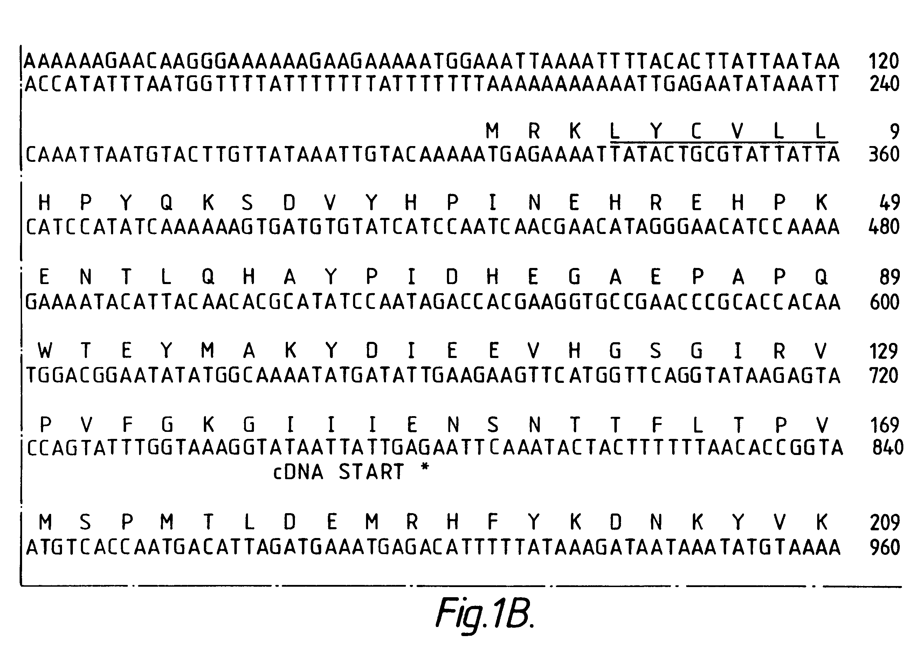

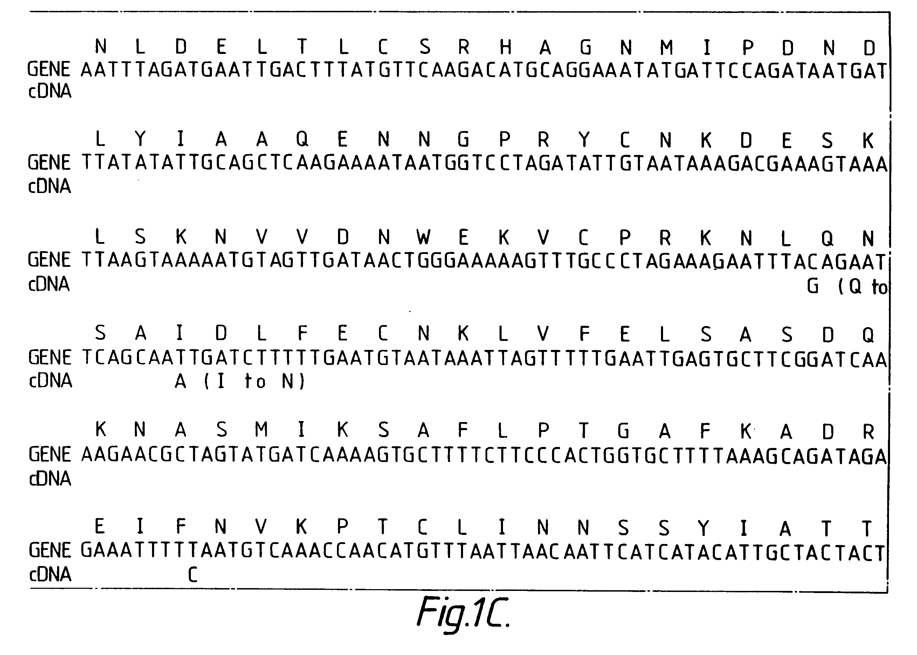

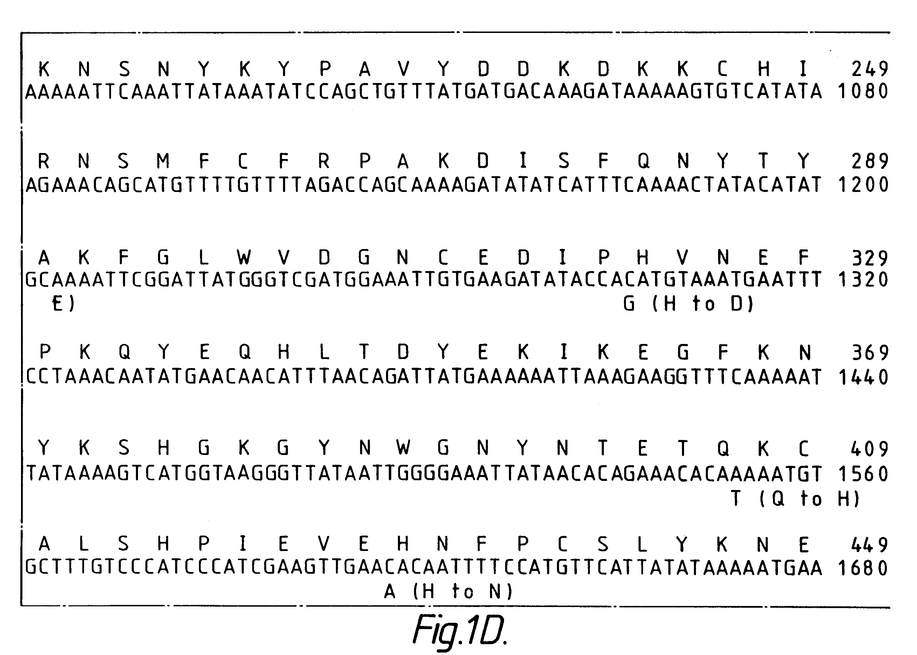

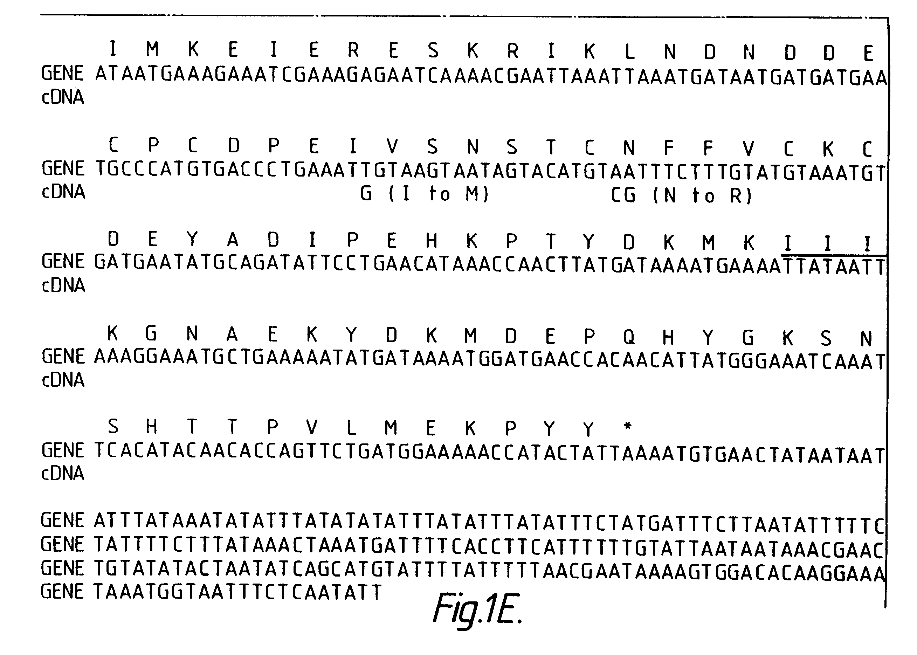

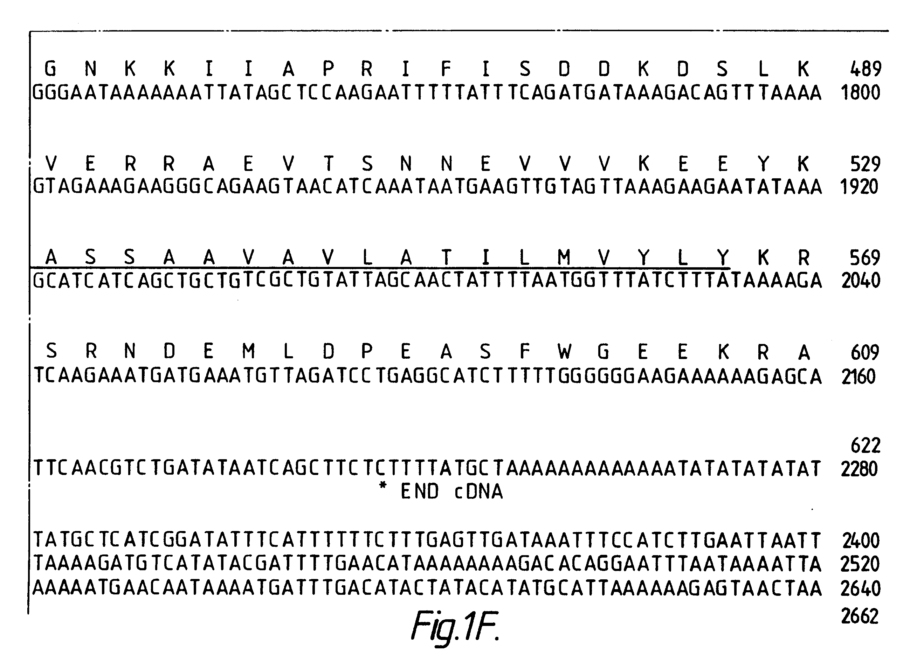

Preferably, the antigen is a polypeptide having a primary structure which includes the amino acid sequence set out in Figure 1, or an antigenic fragment thereof.

The invention also provides a method for actively immunizing a host against Plasmodium falciparum which method comprises administering to the host an antigen according to the present invention, or an antigenic fragment thereof.

The invention provides a vaccine comprising an antigen of the present invention, or an antigenic fragment thereof, a pharmaceutically acceptable carrier or diluent, and optionally an adjuvant.

The present invention also extends to a recombinant DNA molecule comprising all or a portion of a nucleotide sequence which is capable of being expressed as a polypeptide having the antigenicity of an antigen as described above, or an antigenic fragment thereof, or a recombinant cloning vehicle or vector, or a host cell comprising a said recombinant DNA molecule. In this aspect, preferably the recombinant DNA molecule, recombinant cloning vehicle or vector, or host cell comprises all or a portion of a nucleotide sequence as set out in Figure 1.

Finally, the invention extends to a synthetic polypeptide prepared by expression of all or a portion of a nucleotide sequence as described above, and to a vaccine composition comprising a said synthetic polypeptide.

The present invention relates to a previously unrecognized rhoptry antigen that has the solubility characteristics and primary structural features of an integral membrane protein. This antigen, which has been designated rhoptry membrane antigen-1 (RMA-1), appears to be transported to the merozoite surface in late schizonts and free merozoites. The sequence of RMA-1 has been highly conserved between two isolates of P.falciparum and between P.falciparum and the murine malaria P.chabaudi.

Further features of the present invention will become apparent from the detailed description in the following Example, and the accompanying Figures.

In the Figures:
Figure 1 shows the nucleotide sequence of the RMA-1 gene of P.falciparum isolate D10 (top line) and a RMA-1 cDNA from isolate NF7 (bottom line, the asterisks denote the extent of the sequence). The predicted polypeptide sequence is translated. The cDNA sequence is only shown where it differs from the genomic sequence. Where this also results in an amino acid change, the change is shown in brackets. Two stretches of hydrophobic amino acids are underlined. Cysteine residues are shown in bold.

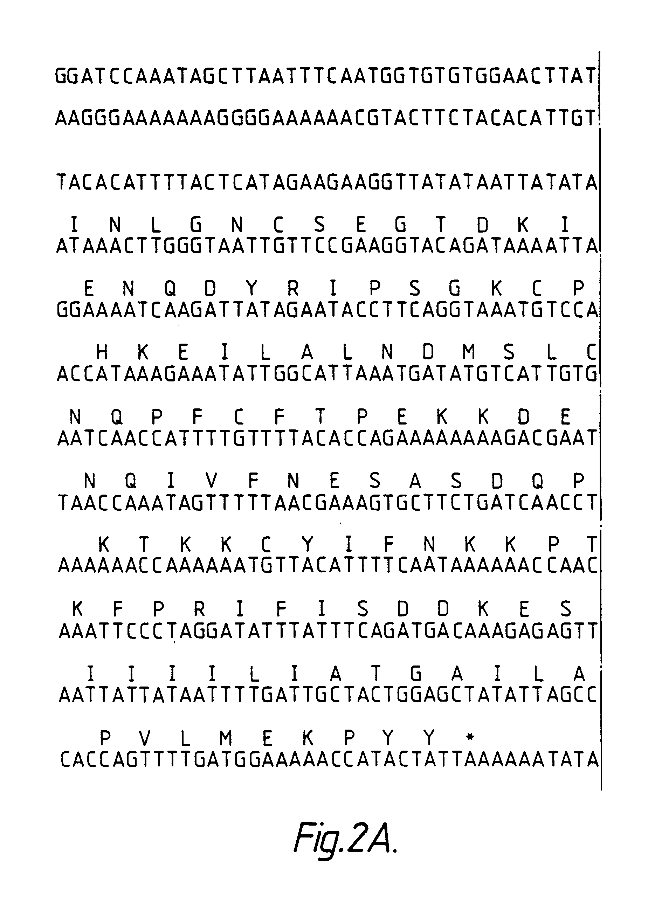

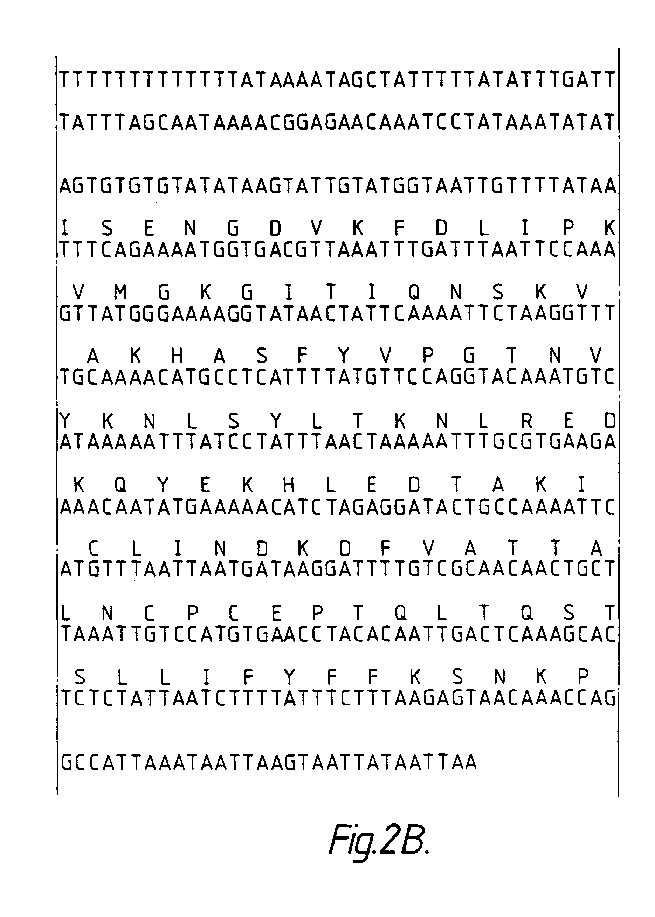

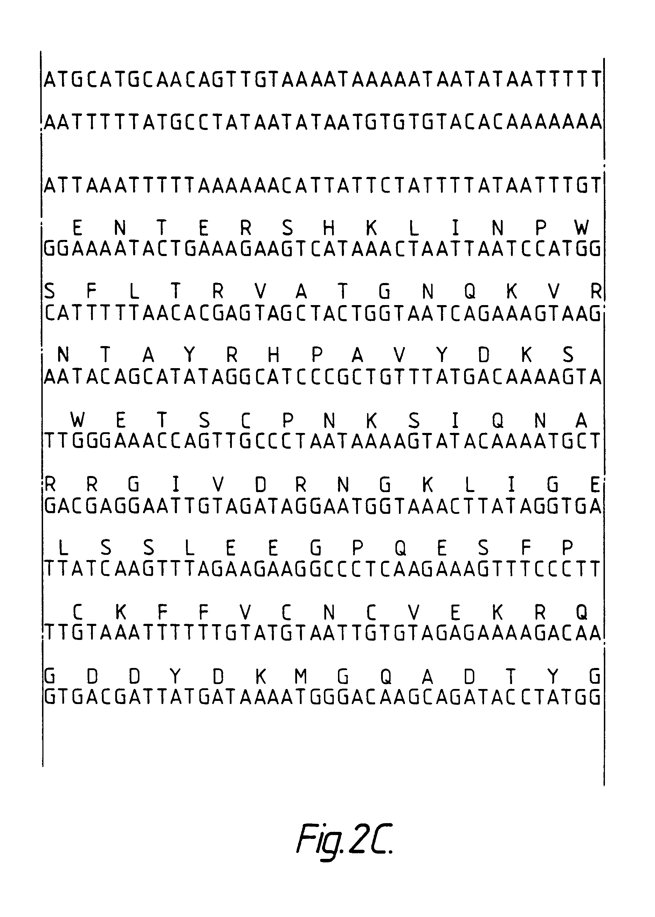

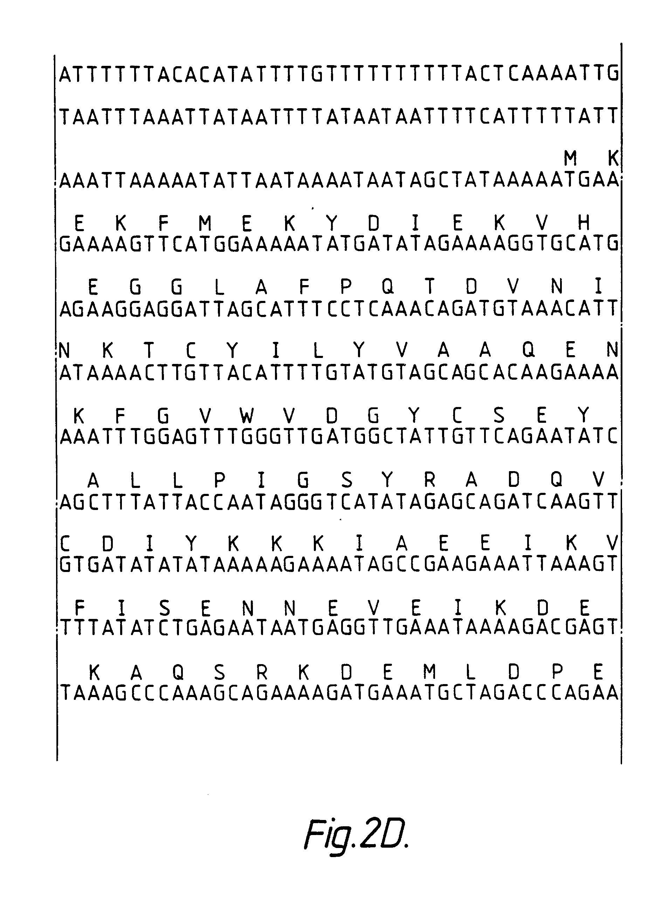

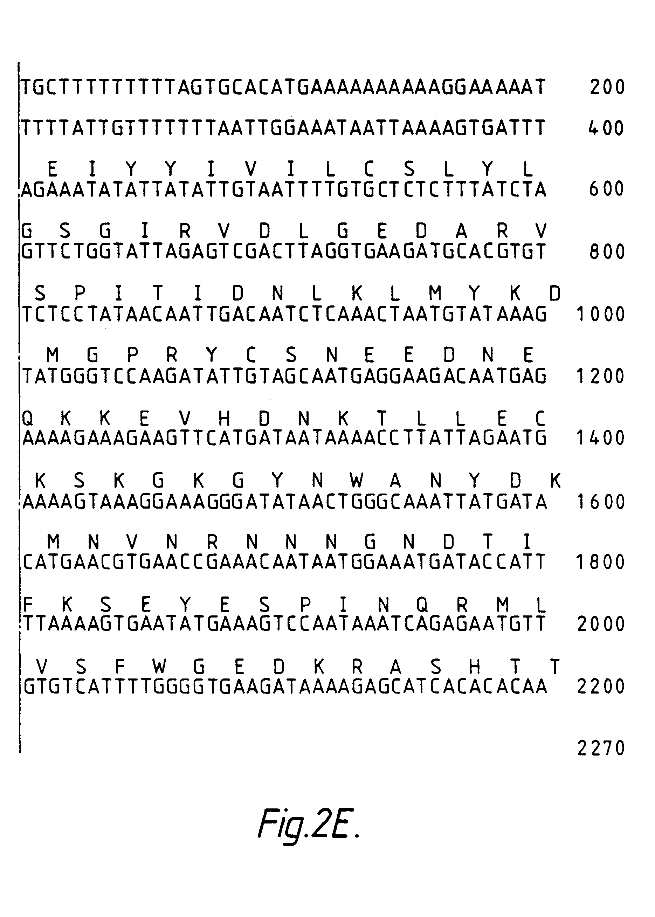

Figure 2 shows the nucleotide sequence of the RMA-1 gene of P.chabaudi adami DS. The predicted polypeptide sequence is translated. Two stretches of hydrophobic residues are underlined.

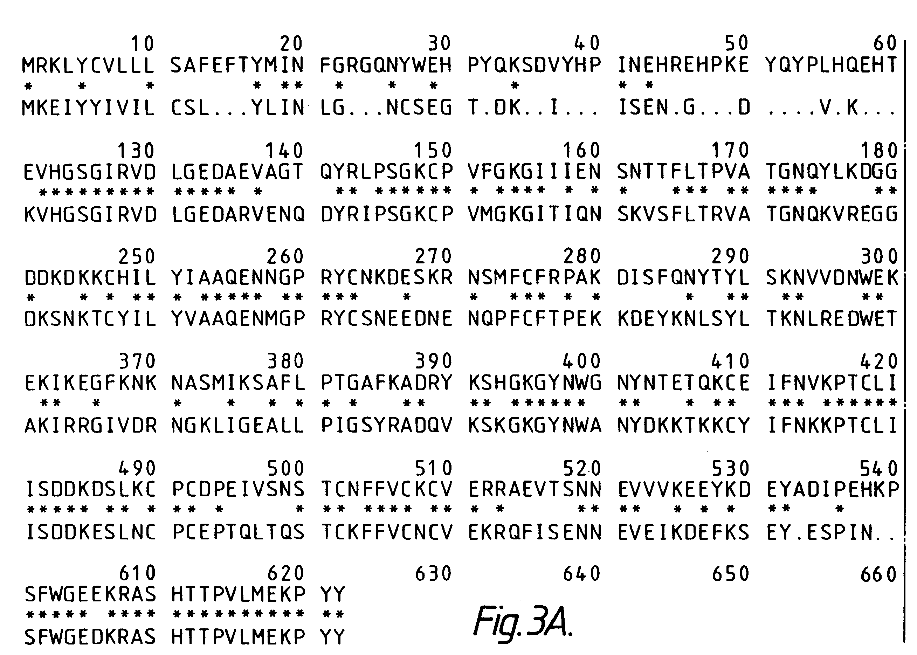

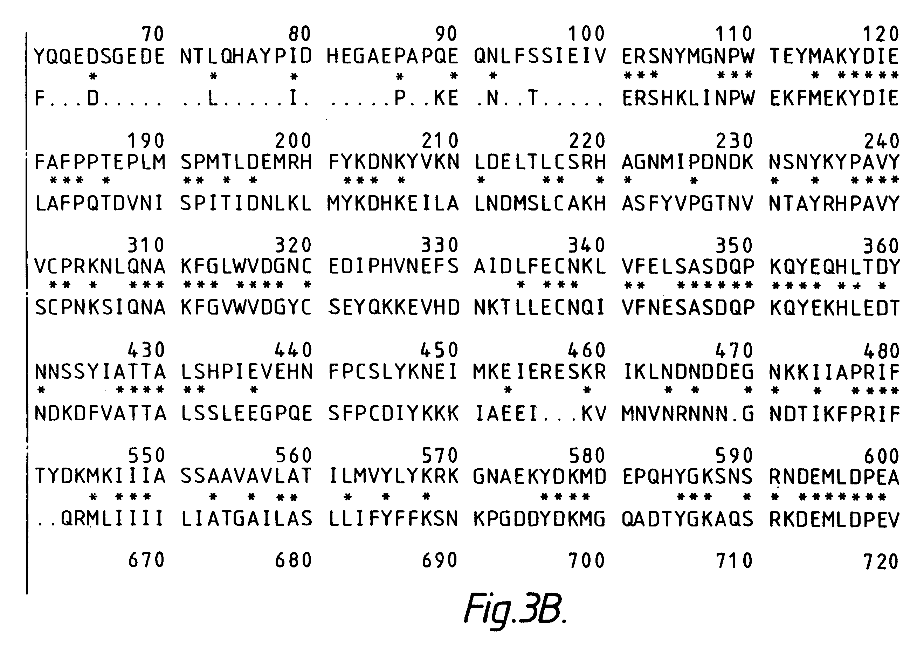

Figure 3 is a comparison of the predicted amino acid sequences of RMA-1 from P.falciparum isolate D10 (top) and P.chabaudi adami DS (bottom). Asterisks denote amino acid identities and dots denote gaps in the sequence.

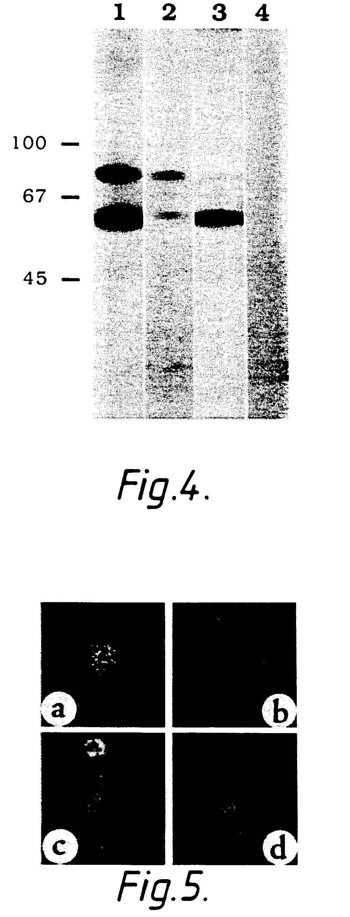

Figure 4 shows the extraction of RMA-1 into Triton X-114. When total infected erythrocytes (A) were fractionated by SDS-PAGE, electroblotted to nitrocellulose and the immunoblots probed with affinity purified rabbit antibodies to the fusion protein produced by clone Ag352.24, two polypeptides were detected: one of M, 80,000 and the other of M, 62,000. Infected erythrocytes were fractionated by temperature-dependent phase partitioning with the detergent Triton X-114 to generate three fractions: aqueous phase (B) in which the M, 80,000 polypeptide was relatively enriched; Triton X-114 phase (C) in which the M, 62,000 polypeptide was relatively enriched; and insoluble pellet (D) free of either of the RMA-1 polypeptides.
Figure 5 shows indirect immunofluorescence microscopy with antibodies to RMA-1. Antibodies affinity purified on the fusion protein produced by Ag352.24 from the serum of a rabbit immunized with this fusion protein were used to label erythrocytes containing various asexual blood stages of P.falciparum isolate FC27: (a) rhoptry fluorescence on a mature schizont; (b) and (c) rhoptry and merozoite surface labelling; (d) surface labelling on mature merozoites.

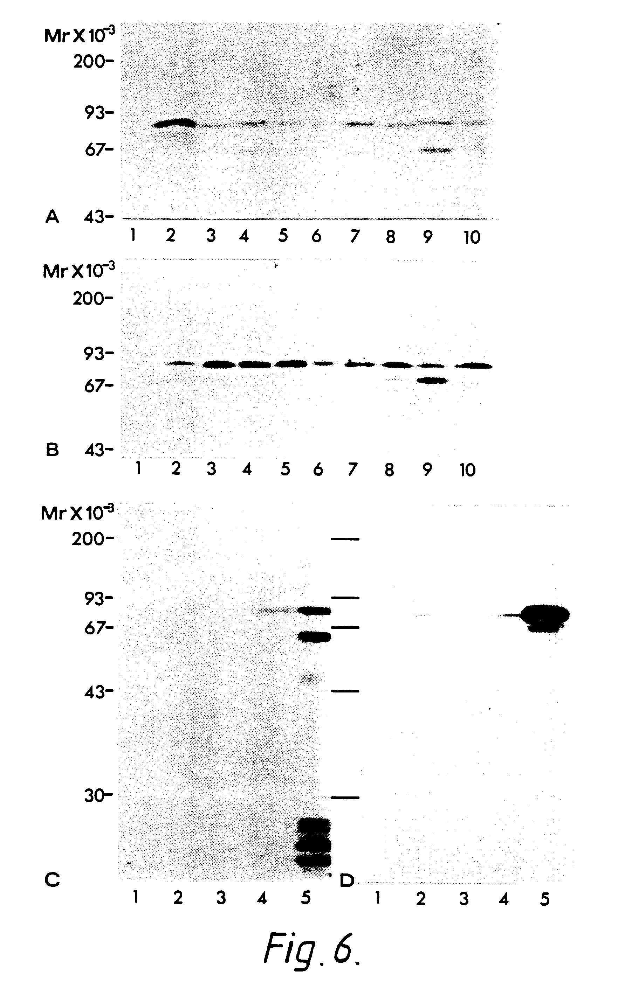

Figure 6 shows the strain and stage specificity of RMA-1. To examine strain specificity erythrocytes infected with nine different isolates of P.falciparum were fractionated by SDS-PAGE, electroblotted to nitrocellulose and probed with rabbit affinity purified antibodies to the fusion protein produced by clone Ag352.24 (A) or the anti-QF3 monoclonal antibody 7H850 (B). Lane 1, uninfected erythrocytes; lane 2, NF7; lane 3, K-1; lane 4, FC27; lane 5, V1; lane 6, FC27 clone D10; lane 7, FC27 clone E12; lane 8, ITG2; lane 9, NF54 clone 3D7; lane 10, CSL2.
   To examine stage specificity different asexual blood stages of isolate FC27 were fractionated by SDS-PAGE, electroblotted to nitrocellulose and probed with rabbit affinity purified antibodies to the fusion protein produced by clone Ag352.24 (C) or the anti-QF3 monoclonal antibody 7H850 (B). Lane 1, uninfected erythrocytes; lane 2, ring-stage trophozoites; lane 3, mature trophozoites; lane 4, schizonts; lane 5, merozoites.

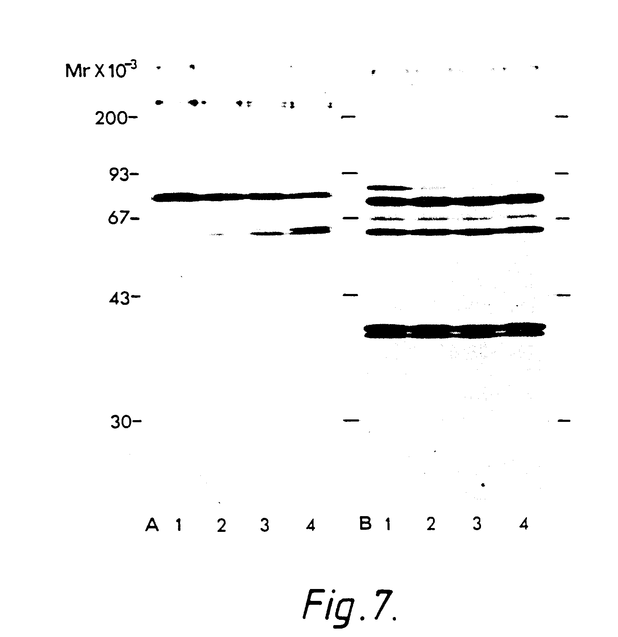

Figure 7 shows the processing of RMA-1. Pulse-chase experiments were carried out in which mature stages of P.falciparum isolate FC27 were labelled with ³⁵ S-methionine for 15 minutes and then cultured in the absence of ³⁵ S-methionine for 0 minutes (lane 1); 15 minutes (lane 2); 30 minutes (lane 3); 60 minutes (lane 4). The radiolabelled parasites were processed for immunoprecipitation with either rabbit anti-Ag352.24 (A) or the QF3 monoclonal antibody 7H850 (B) as described in the Materials and Methods. The immunoprecipitations were fractionated by SDS-PAGE and the labelled antigens visualized by autoradiography.

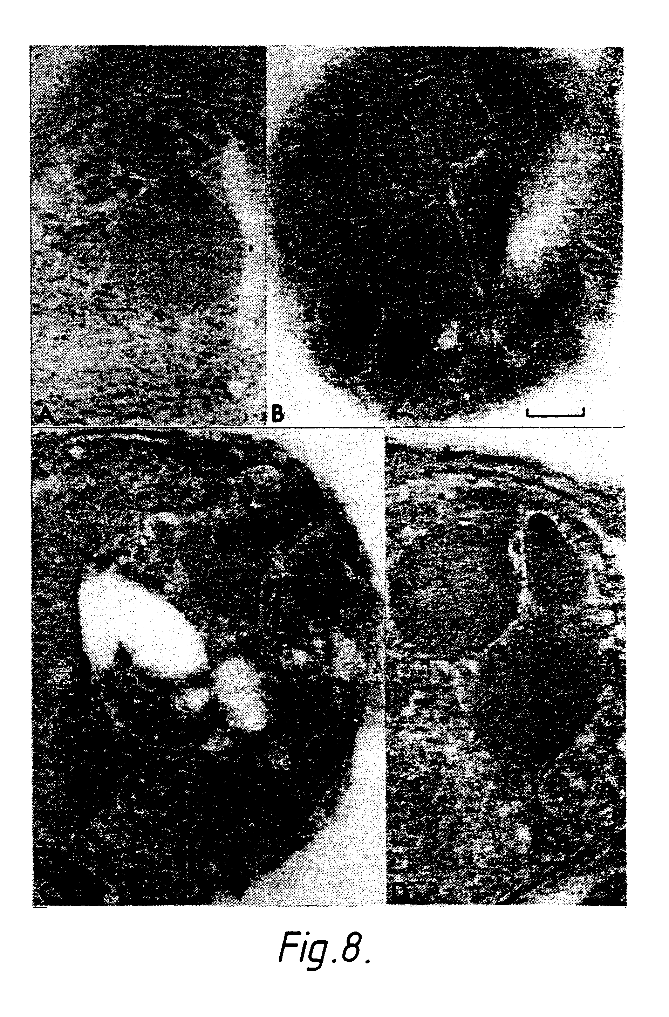

Figure 8 shows the localization of RMA-1 by immunoelectronmicroscopy.Post-embedding labelling of sections of P.falciparum isolate FC27 with human anti-352 antibodies (A,B,C) or with rabbit anti-QF3 antibodies (D), followed by protein A-5nm gold. Labelling occurred over the neck of the rhoptries with anti-352 antibodies and over the rhoptry body for anti-QF3 antibodies.

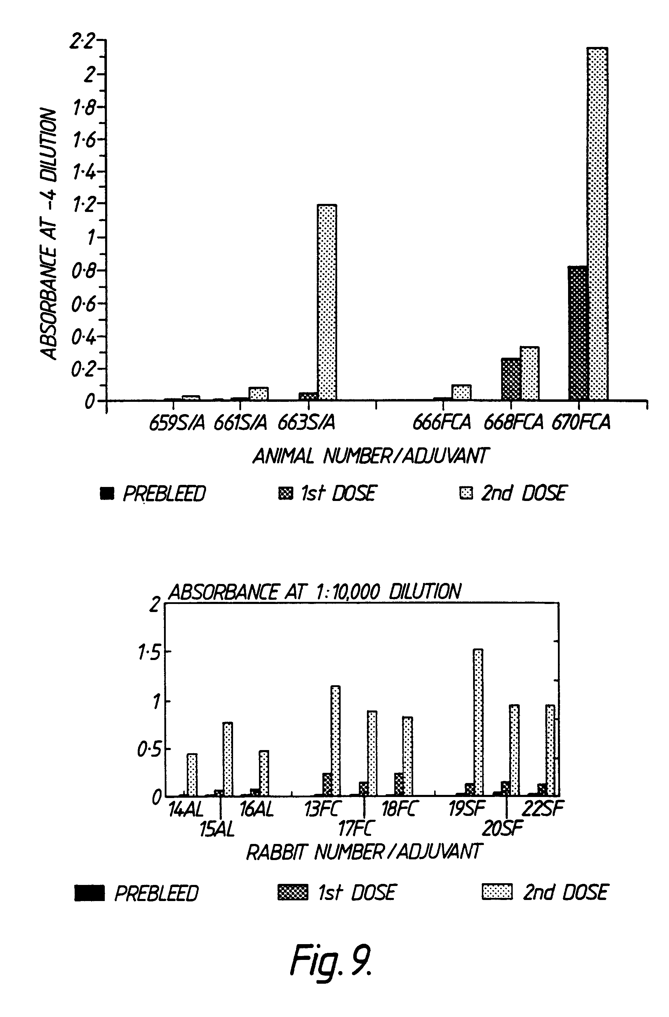

Figure 9 shows the immunogenicity of recombinant RMA-1. ELISA readings on individual rabbit sera, from rabbits designated by number under the graph, are shown. At the top, the responses to the GST- fusion protein are shown. At the bottom, the responses to RMA-1 expressed in baculovirus are shown. Adjuvants used were: AL = alum (aluminium phosphate); FC (or FCA) = Freund's complete adjuvant; S/A = squalene/Arlacel A (Ciba Geigy); SF = SAF-1 (Syntex).

### EXAMPLE

### Material and Methods

Parasites: P.falciparum isolate FCQ27/PNG (FC27), was obtained through collaboration with the Papua New Guinea Institute of Medical Research. D10 and E12 are cloned lines of FC27 (Anders et.al. 1983). The origins of P.falciparum isolates NF7, K1, V1, ITG2, 3D7 and CSL2 have been described (Peterson et.al., 1988). Parasites were maintained in culture as described by Trager and Jensen (1976).
   Antisera: Monoclonal antibody 7H850, specific for the QF3 antigen and rabbit antisera raised by immunization with QF3 antigen affinity-purified on the monoclonal antibody were obtained from the Queensland Institute of Medical Research. Production of the monoclonal antibody and purification of QF3 antigen are described elsewhere (Schofield et.al., 1986).
Rabbits were immunized with the fusion polypeptide produced by the clone pGEX-2T 352.24 (Ag352.24), and affinity-purified on glutathione-agarose as described (Smith and Johnson, 1988).
Human and rabbit antibodies were affinity-purified on adsorbents prepared by coupling the Ag352.24 fusion protein to CNBr-activated Sepharose as described (Crewther et.al., 1986).
   Immunoelectronmicroscopy: Post-embedding immunolabelling was performed as described in Culvenor et.al. (1986). Cultured parasites were fixed for ten minutes in 0.25% glutaraldehyde, dehydrated in 70% ethanol and embedded in L.R. white resin. Thin sections were incubated on droplets of antibody for 1 hr, washed, then incubated with protein A-5nm colloidal gold (Janssen) and stained with uranyl acetate.

### Radiolabelling of parasites and immunoprecipitation:

Routinely, parasites were synchronized once with sorbitol (Lambros and Vanderberg, 1979), mature trophozoites and schizonts were washed once in methionine-free RPMI 1640 medium supplemented with 10% human serum and labelled for two hours at 37°C in the same medium with ³⁵S-methionine at 200µCi/ml. Similarly, mature parasites were label led with L-[2,3,5,6-³H]tyrosine, L-[2,5-³H]histidine, L-[2,3,4,5-³H]proline or L-[4,5-³H]lysine monohydrochloride in the appropriate amino acid-free medium at 100µCi/ml or with [p9,1 0(n)-³H]myristic acid in regular medium at 100µCi/ml.

For pulse-labelling experiments, parasites were synchronized twice with sorbitol at an interval of 33 hours so that at the start of the experiment only ring-stage parasites 0-4 hours old were present. Aliquots of these synchronous parasite cultures were labelled for 30 mins with 60µCi/ml ³⁵ S-methionine at 1,10,20,26,29,32, 35,38,41,44 and 48 hours after the second synchronization. After labelling, cells were washed twice in serum-free RPMI 1640 with 1.7mM cold methionine, and the pellets snap frozen. Duplicate cultures were harvested, washed three times in serum-free RPMI 1640 and smeared for subsequent immunofluorescence assay.

In general, pulse-chase labelling experiments were performed with parasites that had received two sorbitol treatments resulting in a 4-5 hour spread of maturation. These synchronous parasites wre labelled with 100µCi/ml ³⁵ S-methionine for 15-45 minutes at 37°C. After the labelling period, the cells were harvested and an aliquot removed, washed twice in serum-free RPMI 1640 containing 1.7mM cold methinine and the cell pellet snap frozen. The remaining culture was washed twice in complete medium with 1.7mM cold methionine and incubated at 37 _{°} C in this same medium. Further aliquots were subsequently harvested, as described above, after various time intervals. Giemsa-stained smears were examined at each time point confirmed the stage of maturation of the parasites. Frozen cell pellets were extracted for 30 mins at 4 _{°} C in at least ten volumes of NaCI-EDTA-Tris buffer supplemented with Triton X-100 (T-NET) as described by Kessler (1975) containing a cocktail of protease inhibitors comprising 4mM phenylmethylsulphonyl fluoride (Sigma Chemical Co.), 5mM iodoacetamide (BDH), 1mM L-1-tosylamide-2-phenylethylch- loromethyl ketone (Sigma Chemical Co.), 1 mM N-a-p-tosyl-L-lysine chloromethyl ketone (Sigma Chemical Co.), 25µg/ml leupeptin (hemisulphate salt) (Sigma Chemical Co.), 25µg/ml antipain (dihydrochloride) (Sigma Chemical Co.), 25µg/ml chymostatin (Sigma Chemical Co.) and 25µg/ml pepstatin (Sigma Chemical Co.). Insoluble material was removed by centrifugation at 10,000g for 10 mins at 4 _{°} C. The labelled extracts were precleared using formalin-fixed Staphylococcus aureus Cowan 1 strains (Commonwealth Serum Laboratories) and Sepharose 4B beads (Pharmacia) for 30 min at 4 _{°} C. Preclearing agents were removed by centrifugation for 5 min at 5,000g at 4 _{°} C. Specific antisera were added to aliquots of the labelled extracts and incubated at 4°C for 2-4 hours. Immune complexes were precipitated by the addition of Protein A-Sepharose 4B (Pharmacia). The beads were washed four times with T-NET containing protease inhibitors. Culture supernatants collected after labelling of parasites were centrifuged for 1 hour at 100,000g prior to immunoprecipitation.
SDS-PAGE: SDS-PAGE was performed essentially as described by Laemmli (1970). Immunoblotting was carried out as described (Crewther et.al., 1986).

### Molecular cloning and DNA sequencing;

The construction and screening of the P.falciparum NF7 cDNA library in λgt11-Amp3 has been described previously (Stahl et.al. 1984). The P.falciparum partial Sau4AI genomic library in EMBL3 was made as follows: A partial Sau3AI digest of D10 genomic DNA was size fractionated (10-20 kb) on an agarose gel and ligated into BamHl digested, dephosphorylated XEMBL3. The resulting clones were screened with the NF7 cDNA clone, Ag352, using standard procedures (Maniatis et.al. 1982). The P.falciparum partial Sspl genomic library in λgt10 was made as follows: A partial Sspl digest of P.falciparum D10 genomic DNA was methylated with EcoRl methylase, ligated with EcoRl linkers and digested with EcoRl. Excess linkers were removed by size-fractionation on an agarose gel and the fragments were ligated into EcoRl digested, dephosphorylated λgt10. The resulting clones were screened with the NF7 cDNA clone, Ag352. The P.chabaudi adami DS sheared genomic library in λgt10 was made as follows: P.chabaudi adami DS genomic DNA was sheared through a 26 gauge needle, methylated with EcoRl methylase and ligated with EcoRl linkers. Excess linkers were removed and the fragments were size-fractionated (4-7 kb) on an agarose gel, then ligated into EcoRl digested, dephosphorylated λgt10. The resulting clones were screened with the NF7 cDNA clone, Ag352. Positively hybridizing clones were plaque purified through successive rounds of screening, XDNA prepared and the inserts isolated and subcloned using standard procedures (Maniatis et.al. 1982). DNA inserts were further subcloned into M13 vectors (Messing and Vieira, 1982) and sequenced using the chain termination method (Sanger et.al. 1977).

### Expression of the C-terminal 52 amino acids of RMA-1 in E.coli:

The C-terminal 52 amino acids of RMA-1 were expressed in E.coli using the pGEX-2T plasmid vector described by Smith and Johnson (1988). The partially complementary oligonucleotides 5'GATCCGGAAAT-GCTGAAAAATATGATAAAATGGATGAACCACAAC and 5'TAATGTTGTGGTTCATCCATTTTATCATATTTT-TCAGCATTTCCG which encode the complementary strands of the 15 amino acids C-terminal to the transmembrane anchor region where annealed then ligated with a Fokl to EcoRl (linker) fragment (nucleotides 2083-2255, Fig. 1) from clone NF7, and BamHl and EcoRl digested pGEX-2T. The ligation was transformed into E.coli JPA101 and the correct recombinant selected. This clone is designated Ag352.24.

### Expression of near-full length RMA-1 in E.coli:

A segment of RMA-1 consisting of the end filled HinPl to Rsal site nucleotides 366 to 1833 (from Fig.1) was inserted into the filled BamHl site of Ag352.24, transformed and selected as above. This clone is designated Ag352.2.

### Expression of RMA-1 in Baculovirus:

The full length RMA-1 gene was removed as a BamHl/BgIII fragment (nucleotides 327-2247 in Fig.1) from a plC20H subclone (Marsh et.al., 1984) and was inserted into the unique BamHl site of the pAcRP23 (provided by Dr.R.Possee) baculovirus transfection vector behind the baculovirus polyhedrin gene promoter. The pAcRP23/Ag352 DNA was mixed with wild type baculovirus DNA and transfected into Sf9 insect cells. Plaques resulting from the transfection were screened microscopically and those lacking polyhedra were subjected to two further rounds of plaquing. A number of the resultant polyhedron-negative plaques were screened for the presence of the Ag352 gene and all proved to be positive. Virus from recombinant plaques (designated baculovirus 352) was used to infect fresh cultures of Sf9 cells and production of Ag352 by infected cells examined by SDS-PAGE, Western blot and immunofluorescence.
Immunogenicity: Rabbits were immunized with 1.0ml of antigen formulation I/M in the left back leg. A second dose was given in the right back leg, four weeks after the first dose. Bleeds were taken on the day of immunization and at two weekly intervals, except that the final bleed was taken 7-8 days after the second dose. For animals in which squalene/Arlacel A (Ciba Geigy nor-MDP formulation) was used as adjuvant, 100µg nor-MDP was used/dose. All antigens were at 200µg/dose.

### RESULTS

### Structure of RMA-1 and its gene in P.falciparum.

Screening a \gt11-Amp3 cDNA clone library (NF7) with human anti-malarial antibodies (Stahl et.al. 1984) identified a novel cDNA clone designated Ag352. The DNA sequence of Ag352 is shown in Figure 1. To complete the coding sequence, two overlapping genomic fragments of isolate FC27 (a 5' partial Sau3A and a 3' partial Sspl fragment) were cloned and sequenced (Figure 1).

The FC27 genomic sequence extends the 1438 nucleotide NF7 cDNA sequence a further 514 nucleotides 3' and 809 nucleotides 5'. A single long open reading frame is present in the FC27 genomic sequence between nucleotides 333 to 2202 encoding a polypeptide of 622 residues with a predicted molecular weight of 71,929 daltons. Based on the subcellular localization results presented below, this polypeptide was termed rhoptry membrane antigen-1 (RMA-1).

The FC27 genomic and the NF7 cDNA sequences are highly homologous with 9 nucleotide differences in 1438 nucleotides and 7 amino acid differences. Strikingly, only 1 nucleotide difference is silent and 5 of the amino acid differences result in charge changes. These data indicate strong selection, perhaps immunological, on the polypeptide sequence.

Comparison of the sequence of RMA-1 with the Genebank and NBRF databases failed to identify any similar proteins. However, the RMA-1 polypeptide sequence has a number of striking features. Unlike most other blood stage antigens of P.falciparum, RMA-1 lacks repetitive sequences. The antigen has the primary structure expected for an integral membrane protein. It contains 2 hydrophobic stretches, one near the N-terminus, presumably part of a signal peptide, and a second located 55 amino acids from the C-terminus. This consists of 21 predominantly hydrophobic residues and lacks charged residues. It is preceded by a lysine residue and followed by the tripeptide Lys-Arg-Lys, consistent with this region being a membrane spanning domain.

All 17 cysteine residues precede the predicted membrane-spanning domain. The most N-terminal cysteine is presumably removed along with the signal peptide. The remaining 16 residues could form intramolecular disulphide bonds and therefore could be critical for maintaining the secondary structure of the molecule. Consistent with this idea, the mobility of RMA-1 was decreased when electrophoresed under reducing conditions compared to non-reducing conditions (data not shown). The importance of the cysteine residues is supported by the observation that all 16 are found conserved in RMA-1 from P.chabaudi (see below).

### Structure of RMA-1 and its gene in P.chabaudi.

A P.chabaudi adami DS sheared genomic library in \gt10 was screened with the P.falciparum NF7 cDNA clone (described above) to identify the homologous gene in this species. The DNA sequence of the hybridizing region of a positive clone is shown in Figure 2. A single long open reading frame is present between nucleotides 556 and 2229 encoding a polypeptide of 558 amino acids with a predicted molecular weight of 63,901 daltons.

The predicted polypeptide of RMA-1 from P.chabaudi and P.falciparum show a remarkable degree of similarity. Like the P.falciparum homologue, the P.chabaudi RMA-1 polypeptide sequence predicts 2 hydrophobic stretches (underlined in Figure 2) located in very similar positions. These presumably act as an N-terminal signal sequence and a trans-membrane anchor. The sequence lacks repeats, and the 16 cysteine residues, which were such a striking feature of the (predicted) mature P.falciparum molecule are all found conserved in the P.chabaudi molecule. The overall amino acid sequence homology between the 2 species is 43% (Figure 3).

### RMA-1 partitions into Triton X-114.

When cell extracts are fractionated by temperature dependent phase partitioning with the non-ionic detergent Triton X-114, integral membrane proteins are recovered in the detergent phase. The solubility characteristics of RMA-1 were examined using this procedure. Parasite cell pellets from isolate FC27 were solubilized in Triton X-114 and separated into an aqueous phase, a Triton X-114 phase and an insoluble pellet and fractionated by SDS polyacrylamide gel electrophoresis. An immunoblot of these fractions was probed with antibodies affinity purified (Crewther et.al., 1986) on a glutathione S-transferase fusion protein (Smith and Johnson, 1988) containing only the C-terminal 52 amino acids (571 to 622, Figure 1, Ag352.24). The antibodies reacted with two polypeptides of M, 80,000 and 62,000 which were present in variable amounts in the total parasite material, in the aqueous phase and in the Triton X-114 phase (Figure 4). The M, 62,000 polypeptide appears to be a processed fragment of the M, 80,000 (see below). Both the M, 80,000 and 62,000 polypeptides are partially soluble in Triton X-114, the smaller fragment being more soluble probably because it has lost hydrophilic N-terminal residues.

### RMA-1 is localized to the rhoptries and the merozoite surface.

Indirect immunofluorescence microscopy with affinity-purified human antibodies (described above) stained mature asexual blood forms. Segmented schizonts showed a punctate pattern of immunofluorescence within merozoites (Figure 5a) characteristic of antigens located in the rhoptries (Holder et.al. 1985). This pattern was also evident within some merozoites of disrupted schizonts although other clusters of merozoites gave a "bunch of grapes" fluorescence pattern, characteristic of a merozoite surface location (Holder et.al. 1982) (Figure 5d). Patterns of fluorescence intermediate to these were also evident in some mature schizonts (Figure 5b and 5c) where there was merozoite surface fluorescence with "hot-spots" at the apex of each merozoite.

### RMA-1 is present in all isolates of P.falciparum

Nine isolates of P.falciparum of diverse geographic origins were examined by immunoblotting for reactivity with antibodies to RMA-1. Asynchronous parasite preparations solubilized in SDS sample buffer and fractionated on 10% SDS-polyacrylamide gels were probed with affinity purified rabbit anti-Ag352.24 antibodies that had been pre-adsorbed on the Triton X-100-insoluble fraction of normal human erythrocytes. In each isolate M, 80,000 and 62,000 polypeptides, sometimes resolved as doublets were identified (Figure 6a). In some isolates there was weak reactivity with an additional polypeptide of M, 70,000.

A previously identified rhoptry antigen, QF3, has polypeptide components of similar size to the two polypeptides recognized by anti-Ag352.24 antibodies. The monoclonal antibody 7H850, which recognizes QF3 (Schofield et.al. 1986; Bushell et.al. 1988), when used to probe an immunoblot of the 9 P.falciparum isolates also recognized polypeptides of -M, 80,000 and M, 62,000 that like the polypeptides recognized by anti-Ag352 antibodies were invariant in size among these isolates (Figure 6b).

### RMA-1 is found in mature asexual blood-stages.

When synchronized parasites were examined by immunoblotting RMA-1 was detected in schizonts and free merozoites but not in rings or other asexual blood-stages (Figure 6c). The QF3 antigen differed from RMA-1 in that it was clearly present in ring stage parasites in addition to schizonts and merozoites (Figure 6d).

In pulse-labelling studies, synthesis of both Ag352 and QF3 antigens was first detected in mature trophozoites, 29-32 hours old. Maximum synthesis of RMA-1 occurred in mature schizonts (39-41 hours) whereas peak synthesis of QF3 occurred in mid-schizonts, 36 hours after the second synchronization (data not shown). No synthesis of either antigen was detected after the 44 hour time point. Consistent with the immunoblotting studies, affinity-purified rabbit anti-Ag352.24 antibodies specifically immunoprecipitated doublets of M, 80,000 and 62,000 from Triton X-100 lysates of mature trophozoites and schizonts labelled with ³⁵S-methionine. The anti-QF3 monoclonal antibody, 7H850, immunoprecipitated Mᵣ 80,000and 62,000 polypeptides (and a minor Mᵣ 70,000 polypeptide) together with a Mᵣ 39,000 and 38,000 doublet. Similar results were obtained when both antibodies were used to immunoprecipitate the relevant antigens from lysates of parasites labelled with tritiated proline, tyrosine, lysine or histidine. There was no marked difference in the intensity of labelling of the two antigens with any of these amino acids (data not shown).

Repeated clearing of ³⁵S-methionine-labelled parasite lysates with antibodies specific for RMA-1 failed to diminish the intensity of bands immunoprecipitated with monoclonal antibodies specific for the QF3 antigen. The same was true for the reverse experiment (data not shown).

### The RMA-1, Mᵣ80,000 polypeptide is rapidly processed to a M,62,000 form.

Pulse-chase experiments utilizing a labelling period of 15 minutes showed that the Mᵣ 80,000 polypeptide of RMA-1 was rapidly processed to the Mᵣ 62,000 form (Figure 7). Approximately 50% of the antigen synthesized in mature schizonts during a 15 minute pulse-label was processed to the lower molecular weight form over a 1 hour chase period. When the fate of this antigen was followed over a longer chase period, the same rapid processing was seen (data not shown). Neither the Mᵣ 80,000 or the 62,000 molecules were detected in new rings after reinvasion. This same pattern of processing was also observed if the antigen was pulse-labelled in mature trophozoites and immature schizonts, indicating that cleavage of the larger molecules is not restricted to a particular life-cycle stage (data not shown). Labelling RMA-1 was not detectable by immunoprecipitation of clarified culture supernatants (data not shown).

In contrast, using the same conditions for pulse-chase experiments, it was found that the QF3 antigen was synthesized as a short-lived precursor molecule of M, 84,000, all of which was processed to the M, 80,000 form over a 1 hour chase period (Figure 7B). Processing of the M, 80,000 molecule to the M, 62,000 form of QF3 was not apparent when mature schizonts were pulse-labelled. However, when mature trophozoites and immature schizonts were pulse-labelled and chased for various times, the bulk of this processing was found to occur in mature schizonts during reinvasion. Only the M, 80,000 molecule of QF3 was carried into rings. None of the molecules of the QF3 complex were detectable by immunoprecipitation of clarified culture supernatants collected after the 12 hour chase period in this experiment.

### RMA-1 and QF3 are in different regions of the rhoptry.

Immunogold labelling studies consistently showed localization of the RMA-1 antigen to the neck of the rhoptries of mature merozoites within schizonts (Figure 8a) and, similarly, in free merozites (Figure 8b and c). Occasional gold particles were found around the periphery of merozoites (Figure 8b and c) and over micronemes which were of similar electron density to the rhoptry neck (Figure 8a). Sections derived from the same resin block of FC27 incubated with rabbit anti-QF3 antibodies showed labelling over the body of the rhoptry, not in association with the neck or the plasma membrane (Figure 8d).

### Expression of RMA-1 in baculovirus;

Baculovirus expressed RMA-1 as a protein doublet of M, 84-87,000 with secondary bands of M, 67-77,000. The protein was recognized on immunoblots of baculovirus RMA-1 infected cells by rabbit antisera to RMA-1 and also by human antibodies. The protein was detectable as a minor band on Coomassie- stained gel (data not shown).

### Immunogenicity of recombinant RMA-1 in rabbits:

To examine the immunogenicity of the near-full length RMA-1 expressed in E.coli (Materials and Methods) and of the RMA-1 expressed in baculovirus (see above), rabbits were immunized with 1 ml of an antigen formulation, boosted after four weeks and bled 7-8 days later (Fig. 9). It is clear from the ELISA readings Freund's complete adjuvant gave, on average, a greater response than the nor-MDP adjuvant, and RMA-1 expressed in baculovirus gave a greater response than when expressed as a fusion protein.

### DISCUSSION

Previous studies have identified two antigen complexes in the rhoptries of P.falciparum merozoites reviewed in Anders (1988). In addition to these antigen complexes at least two other rhoptry antigens have been described, one an M, 55,000 polypeptide with the solubility characteristics of an integral membrane protein (Smythe et.al., 1988) and another, a M, 240,000 molecule which is processed to an Mᵣ 225,000 fragment (Roger et.al., 1988). The results reported here show that RMA-1 is unrelated to any of these antigens and thus represents a previously uncharacterized rhoptry antigen. RMA-1 is initially synthesized as an Mᵣ 80,000 polypeptide which is rapidly processed to an Mᵣ 62,000 form. Coincidentally, two of the major components of the lower molecular weight rhoptry complex (QF3) (Schofield et.al. 1986; Bushell et.al. 1988) have the same relative molecular masses but the different locations within the rhoptry, the different processing pattern seen in pulse-chase experiments, and different co-precipitating proteins indicate that the RMA-1 and QF3 polypeptides are different.

RMA-1 appears to be located first in the rhoptry and then exported to the merozoite surface around the time of schizont rupture. Within the rhoptry RMA-1 appears to be particularly localized in the neck (or peduncle). One other rhoptry antigen, the Mᵣ 240,000 polypeptide described by Roger et.al.(1988) is also in this location but the other rhoptry antigens that have been studied using immunoelectronmicroscopy are located in the body of the rhoptry. The heterogenous distribution of rhoptry contents indicated by these observations has not been explained but it is of some interest that RMA-1, located in the rhoptry neck, is an integral membrane protein whereas the Mᵣ 105,000 component of the high molecular weight rhoptry complex, which is located in the body of the rhoptry (Culvenor et.al., 1986) lacks any structural or solubility characteristics of an integral membrane protein (H.Brown, unpublished results). Thus, as there is good evidence of lipid within the rhoptry secretion it may be that integral membrane proteins are associated with lipid in the rhoptry neck. Presumably RMA-1 becomes associated with the mature merozoite surface after being released, possibly together with lipid, via the apical duct of the merozoite. Consistent with this, others have suggested that some release of rhoptry contents may occur at the time of schizont rupture (Bannister et.al., 1986). The presence of RMA-1 within rhoptries and then apparently associated with the merozoite surface provides strong support for RMA-1 as a potential vaccine component. RMA-1 is a natural immunogen which induces antibodies in individuals infected with P.falciparum.

### REFERENCES:

1. Anders, R.F. et.al. (1983), Proc.Natl.Acad.Sci.USA 80: 6652-6656.
2. Anders, R.F. (1988). In: The Biology of Parasitism. (Eds.P.T.Englund and A.Sher), pp.201-204. Alan
R.Liss Inc., New York.
3. Bannister, L.H. et.al. (1986). Parasitology 92:291-303.
4. Braun-Breton, C. et.al. (1986). Molec.Biochem. Parasitol. 20:33-43.
5. Bushell, G.R. et.al. (1988). Molec.Biochem. Parasitol. 28:105-112.
6. Campbell, G.H. et.al. (1984). Am.J.Trop.Med.Hyg. 33:1051-1054.
7. Cooper, J.A. et.al.(1988). Mol.Biochem. Parasitol. 29: 251-260.
8. Crewther, P.E. et.al. (1986). J.lmmunol. Methods. 86:257-264.
9. Culvenor, J.G. et.al. (1986). Exp.Parasitol. 63:58-67.
10. Holder, A.A. and Freeman, R.R. (1981). Nature 294: 361-364.
11. Holder, A.A. and Freeman, R.R. (1982). J.Exp.Med. 156:1528-1538.
12. Holder, A.A. et.al. (1985) Molec.Biochem. Parasitol. 14:293-303.
13. Howard, R.F. et.al. (1984). Am.J.Trop.Med.Hyg. 33: 1055-1059.
14. Kessler, S.W. (1975). J.lmmunol. 117:1482.
15. Laemmli, J.K. (1970). Nature 227:680.
16. Lambros, C. and Vanderberg, J.P. (1979). J.Parasitol. 65: 418-420.
17. Lustigman, S. et.al. (1988) Mol.Biochem. Parasitol. 30:217-224.
18. Maniatis, T. et.al. (1982). Molecular Cloning - A Laboratory Manual. Cold Spring Harbor Laboratory.
19. Marsh, J.L. et.al. (1984). Gene 32:481-485.
20. Messing, J. and Vieira, J. (1982). Gene 19: 269-276.
21. Perrin, L.H. et.al. (1985). J.Clin.lnvest. 75:1718-1721.
22. Peterson, M.G. et.al. (1988). Molec. Cell Biol. 8: 2664-2667.
23. Roger, N. et.al. (1988). Molec.Biochem.Parasitol. 27:135-142.
24. Sanger, F. et.al. (1977). Proc.Natl.Acad.Sci.USA 74:5463-5467.
25. Schofield, L. et.al. (1986). Molec.Biochem. Parasitol. 18:183-195.
26. Siddiqui, W.A. et.al. (1987). Proc.Natl.Acad.Sci. USA 84:3014-3018.
27. Smith, D.B. and Johnson, K.S. (1988). Gene 67:31-40.
28. Smythe, J. et.al. (1988). Proc.Natl.Acad.Sci. USA 85:5195-5199.
29. Stahl, H.D. et.al. (1984). Proc.Natl.Acad.Sci. USA 81:2456-2460.
30. Trager, W. and Jensen, J.B. (1976). Science 193:673- 675.

## Claims

1. A rhoptry membrane antigen of the asexual blood stages of Plasmodium falciparum, which is characterised by:
i) being initially synthesized as a polypeptide having a relative molecular mass of approximately 80,000 as determined by SDS polyacrylamide gel electrophoresis;
ii) being located in vivo in the neck region of the rhoptries of merozoites; and
iii) having the solubility characteristics and a hydrophobic domain typical of an integral membrane protein;
or an antigenic fragment thereof capable of inducing a protective immune response.

2. An antigen fragment as claimed in claim 1 being substantially free from other P.falciparum proteins.

3. An antigen as claimed claim 1 or claim 2 having a primary structure which comprises the amino acid sequence corresponding to the indicated nucleotide sequence: 
GENE GATCTAATAACGGATATATCTATTTTTTTTAAATGCACAAGAAAAAAAAAAAAAAGAAAAAA
ATATTTAATATATTTATATATTATAAAAAAAAGGAAAAAAAAAAAAAAAAAAAAAAATTA

GENE GTAATATATATTTTTATTTTAATATAATTTTTAAAAACCTAATAATTTTATTTTGATATAATTTTTT

L S A F E F T Y M I N F G R G Q N Y W E
GENE TTGAGCGCCTTTGAGTTTACATATATGATAAACTTTGGAAGAGGACAGAATTATTGGGAA

E Y Q Y P L H Q E H T Y Q Q E D S G E D
GENE GAATACCAATATCCATTACACCAGGAACACACATACCAACAAGAAGATTCAGGAGAAGAC

E Q N L F S S I E I V E R S N Y M G N P
GENE GAACAAAATTTATTTTCAAGCATTGAAATAGTAGAAAGAAGTAATTATATGGGTAATCCA

D L G E D A E V A G T Q Y R L P S G K C
GENE GATTTAGGAGAAGATGCTGAAGTAGCTGGAACTCAATATAGACTTCCATCAGGGAAAATGT
cDNA

A T G N Q Y L K D G G F A F P P T E P L
GENE GCTACGGGAAATCAATATTTAAAAGATGGAGGTTTTGCTTTTCCTCCAACAGAACCTCTT
cDNA
 
AAAAAAGAACAAGGGAAAAAAGAAGAAAAAATGGAAATTAAAAATTTTACACTTATTAATAA 120
ACCATATTTAATGGTTTTTATTTTTTTTAAAAAAAAAATTGAGAATATAAATT 240

M R K L Y C V L L 9
CAAATTAATGTACTTGTTATAAATTGTACAAAAATGAGAAAATTATACTGCGTATTATTA 360

H P Y Q K S D V Y H P I N E H R E H P K 49
CATCCATATCAAAAAGTGATGTGTATCATCCAATCAACGAACACATAGGGAACATCCAAAA 480

E N T L Q H A Y P I D H E G A E P A P Q 89
GAAAATACATTACAACACGCATATCCAATAGACCACGAAGGTGCCGAACCCGCACCACAA 600

W T E Y M A K Y D I E E V H G S G I R V 129
TGGACGGAATATATGGCAAAATATGATATTGAAGAAGTTCATGGTTCAGGTATAAGAGTA 720

P V F G K G I I E N S N T T F L T P V 169
CCAGTATTTGGTAAAGGTATAATTATTGAGAATTCAAATACTACTTTTTAACACCGGTA 840
cDNA START *

M S P M T L D E M R H F Y K D N K Y V K 209
ATGTCACCAATGACATTAGATGAAATGAGACATTTTTATAAAGATAATAAATATGTAAAA 960
 
N L D E L T L C S R H A G N M I P D N D
GENE AATTTAGATGAATTGACTTTATGTTCAAGACATGCAGGAAATATGATTCCAGATAATGAT
cDNA

L Y I A A Q E N N G P R Y C N K D E S K
GENE TTATATATTGCAGCTCAAGAAAATAATGGTCCTAGATATTGTAATAAAGACGAAAGTAAA
cDNA

L S K N V V D N W E K V C P R K N L Q N
GENE TTAAGTAAAAATGTAGTTGATAACTGGGAAAAAGTTTGCCCTAGAAAGAATTTACAGAAT
cDNA G (Q to

S A I D L F E C N K L V F E L S A S D Q
GENE TCAGCAATTGATCTTTTTGAATGTAATAAATTAGTTTTTGAATTGAGTGCTTCGGATCAA
cDNA A (I to N)

K N A S M I K S A F L P T G A F K A D R
GENE AAGAACGCTAGTATGATCAAAAGTGCTTTTCTTCCCACTGGTGCTTTTAAAGCAGATAGA
cDNA

E I F 'N V K P T C L I N N S S Y I A T T
GENE GAAATTTTTAATGTCAAACCAACATGTTTAATTAACAATTCATCATACATTGCTACTACT
cDNA C
 
K N S N Y K Y P A V Y Y D D K D K K C H I 249
AAAAATTCAAATTATAAATATCCAGCTGTTTATTATGATGACAAAGATAAAAAGTGTCATATA 1080

R N S M F C F R P A K D I S F Q N Y T Y 289
AGAAACAGCATGTTTTGTTTTAGACCAGCAAAAGATATATCATTTCAAAACTATACATAT 1200

A K F G L W V D G N C E D I P H V N E F 329
GCAAAATTCGGATTATGGGTCGATGGAAATTGTGAAGATATACCACATGTAAATGAATTT 1320
E) G (H to D)

P K Q Y E Q H L T D Y E K I K E G F K N 369
CCTAAACAATATGAACAACATTTAACAGATTATGAAAAAATTAAAGAAGGTTTCAAAAAT 1440

Y K S H G K G Y N W G N Y N T E T Q K C 409
TATAAAAGTCATGGTAAGGGTTATAATTGGGGAAATTATAACACAGAAACACAAAAATGT 1560
T (Q to H)

A L S H P I E V E H N F P C S L Y K N E 449
GCTTTGTCCCATCCCATCGAAGTTGAACACAATTTTCCATGTTCATTATATAAAAATGAA 1680
A (H to N)
 
I M K E I E R E S K R I K L N D N D E
GENE ATAATGAAAGAAATCGAAAGAGAATCAAAACGAATTAAATTAAATGATAATGATGATGAA
cDNA

C P C D P E I V S N S T C N F V C K C
GENE TGCCCATGTGACCCTGAAATTGTAAGTAATAGTACATGTAATTTCTTTGTATGTAAATGT
cDNA G (I to M) CG (N to R)

D E Y A D I P E H K P T Y D K M K I I
GENE GATGAATATGCAGATATTCCTGAACATAAACCAACTTATGATAAAATGAAAATTATAATT
cDNA

K G N A E K Y D K M D E P Q H Y G K S N
GENE AAAGGAAATGCTGAAAAATATGATAAAATGGATGAACCACAACATTATGGAAAATCAAAT
cDNA

S H T T P V L M E K P Y Y *
GENE TCACATACAACACCAGTTCTGATGGAAAAACCATACTATTAAAAATGTGAACTATAATAAT
cDNA

GENE ATTTATAAATATATTTATATATATTTATATTTCTATGATTTCTTAATATATTTTC
GENE TATTTTCTTTATAAACTAAATGATTTTCACCTTCATTTTTGTATTAATAATAAACGAAC
GENE TGTATATACTAATATATCAGCATGTATTTTTATTTTTAACGAATAAAAGTGGACACAAGGAAA
GENE TAAATGGTAATTTCTCAATATATT
 
G N K K I I A P R I F I S D D K D S L K 489
GGGAATAAAAAAATTATAGCTCCAAGAATTTTTATTTCAGATGATAAAGACAGTTTAAAA 1800

V E R R A E V T S N N E V V V K E E Y K 529
GTAGAAAGAAGGGCAGAAGTAACATCAAATAATGAAGTTGTAGTTAAAGAAGAATATAAA 1920

A S S A A V A V L A T I L M V Y L Y K R 569
GCATCATCAGCTGCTGTCGCTGTATTAGCAACTATTTTAATGGTTTATCTTTATAAAAGA 2040

S R N D E M L D P E A S F W G E E K R A 609
TCAAGAAATGATGAAATGTTAGATCCTGAGGCATCTTTTTGGGGGGAAGAAAAAAGAGCA 2160

622
TTCAACGTCTGATATAATCAGCTTCTCTTTTATGCTAAAAAAAAAAATATATATATATAT 2280
* END cDNA

TATGCTCATCGGATATATTTCATTTTTCTTTGAGTTGATAAATTTCCATCTTGAATTAATT 2400
TAAAAGATGTCATATACGATTTTGAACATAAAAAAAGACACAGGAATTTAATAAAATTA 2520
AAAAATGAACAATAAAATGATTTGACATATACTATACATATGCATTAAAAAAAGAGTAAACTAA 2640
2662
 or an antigenic fragment thereof capable of inducing a protective immune response.

4. A recombinant DNA molecule comprising a nucleotide sequence encoding a polypeptide having the antigenicity of an antigen as defined in any one of claims 1 to 3, or an antigenic fragment thereof, capable of inducing a protective immune response.

5. A recombinant DNA molecule as claimed in claim 4 wherein said nucleotide sequence comprises all or a portion of the sequence:

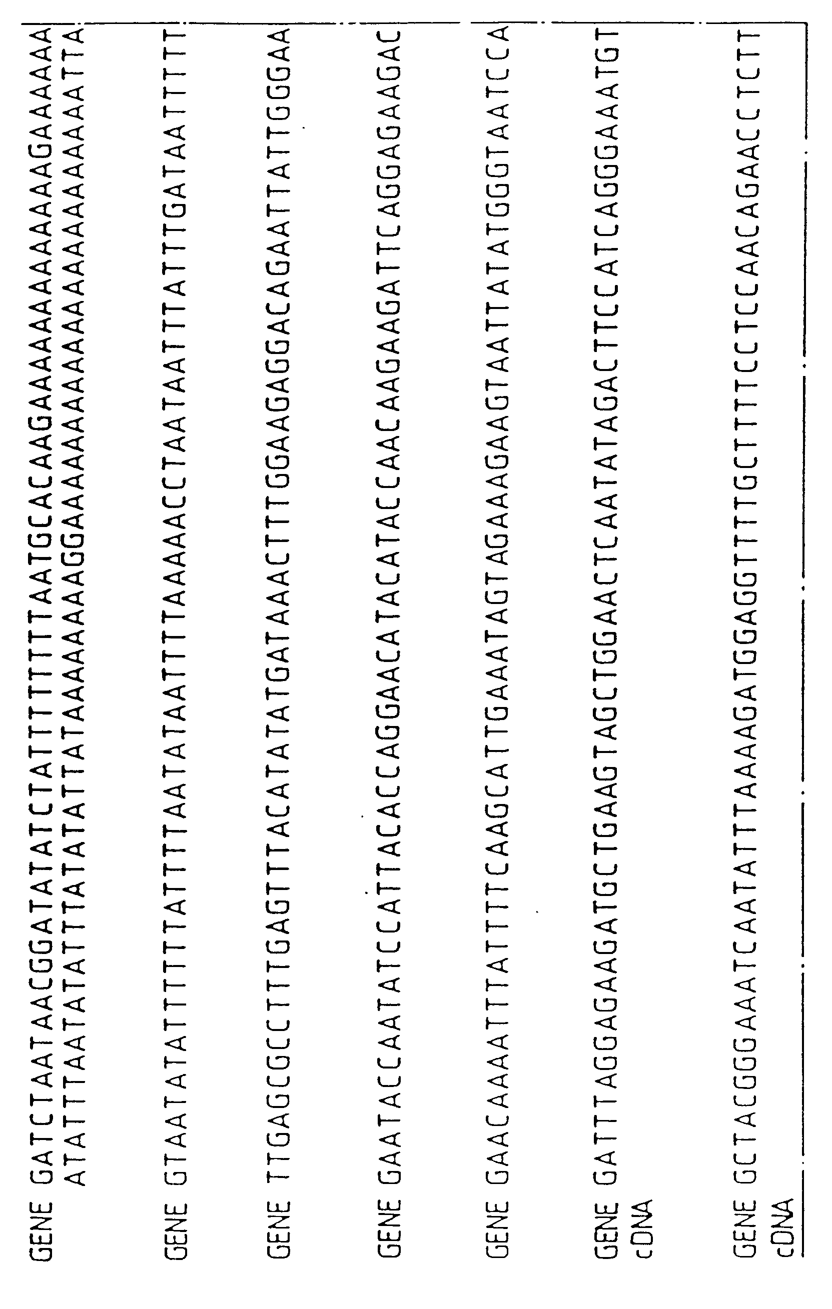
GENE GATCTAATAACGGATATATCTATTTTTTTAATGCACAAGAAAAAAAAAAAAAGAAAAAA
ATATTTAATATATATTTATATATATAAAAAAAAAGGAAAAAAAAAAAAAAAAAAAAATTA

GENE GTAATATATTTTTTATTTTTAATATATAATTTTTAAAAACCTAATAATTTATTTGATAATTTTTT

GENE TTGAGGCCTTTGAGTTTACATATATGATAAACTTTGGAAGAGGACAGAATTATTGGGAA

GENE GAATACCAATATCCATTACACCAGGAACATACATACCAACAAGAAGATTCAGGAGAAGAC

GENE GAACAAAAATTTATTTTCAAGCATTGAAATAGTAGAAAAGAAGTAATTATATGGGTAATCCA

GENE GATTTAGGAGAAGATGCTGAAGTAGCTGGAACTCAATATAGACTTCCATCAGGGAAAATGT
cDNA

GENE GCTACGGGAAATCAATATATTTAAAAGATGGAGGTTTTGCTTTTCCTCCAACAGAACCTCTT
cDNA

AAAAAAGAACAAGGGAAAAAAGAAGAAAAAATGGAAATTAAAAATTTTTACACTTATTATTAATAA 120
ACCATATTTAATGGTTTTATTTTTTTATTTTTTTAAAAAAAAAATTGAGAATATATAAATT 240
9
CAAATTAATGTACTTGTTATAAATTGTACAAAAATGAGAAAAATTATACTGCGTATTATTA 360
49
CATCCATATCAAAAAGTGATGTGTATCATCCAATCAACGAACATAGGGAACATCCAAAA 480
89
GAAAATACATTACAACACGCATATCCAATAGACCACGAAGGTGCCGAACCCGCACCACAA 600
129
TGGACGGAATATATGGCAAAATATGATATTGAAGAAGTTCATGGTTCAGGTATAAGAGTA 720
169
CCAGTATTTGGTAAAGGTATAATTATTGAGAATTCAAATACTACTTTTTAACACCGGTA 840
cDNA START *
209
ATGTCACCAATGACATTAGATGAAATGAGACATTTTATAAAGATAATATAAATATGTAAAA 960

GENE AATTTAGATGAATTGACTTTATGTTCAAGACATGCAGGAAAATATGATGATTCCAGATAATGAT
cDNA

GENE TTATATATTGCAGCTCAAGAAAAATAAATGGTCCTAGATATTGTAATAAAGACGAAAGTAAA
cDNA

GENE TTAAGTAAAAATGTAGTTGATAACTGGGAAAAAGTTTGCCCTAGAAAGAATTTACAGAAT
cDNA                                          G (Q to

GENE TCAGCAATTGATCTTTTTGAATGTAATAAATTAGTTTTTGAATTGAGTGCTTCGGATCAA
cDNA        A (I to N)

GENE AAGAACGCTAGTATGATCAAAAGTGCTTTTCTTCCCACTGGTGCTTTTAAAGCAGATAGA
cDNA

GENE GAAATTTTAATGTCAAACCAACATGTTTAATTAACAATTCATCATACATTGCTACTACT
cDNA        C

249
AAAAATTCAAATTATATAAATATCCAGCTGTTTATGATGACAAAGATAAAAAGTGTCATATA 1080

289
AGAAACAGCATGTTTTGTTTTAGACCAGCAAAAAGATATATCATTTCAAAACTATACATAT 1200

329
GCAAAAATTCGGATTATGGTCGATGGAAATTGTGAAGATATACCACATGTAAATGAATTT 1320
E)                                          G (H to D)

369
CCTAAACAATATGAACAACATTTAACAGATTATGAAAAAATTAAAGAAGGTTTCAAAAAT 1440

409
TATAAAAGTCATGGTAAGGGTTATAATTGGGGAAATTATAACACACAGAAACACAAAATGT 1560
                                                        T (Q to H)

449
GCTTTGTCCCATCCCATCGAAGTTGAACACAATTTTCCATGTTCATTATATAAAAATGAA 1680
                              A (H to N)

GENE ATAATGAAAGAGAAATCGAAAGAGAATCAAAACGAATTAAATTAAAATGATAATGATGATGAA
cDNA

GENE TGCCCATGTGACCCTGAAATTGTAAGTAATAGTACATGTAATTTCTTTGTATGTAAATGT
cDNA G (I to M) CG (N to R)

GENE GATGAATATATGCAGATATTCCTGAACATAAACCAACTTATGATAAAAATGAAAATTATAATT
cDNA

GENE AAAGGAAAATGCTGAAAAATATGATAAAAATGGATGAACCACAACATTATGGGAAATCAAAT
cDNA
*
GENE TCACATACAACACCAGTTCTGATGGAAAAACCATACTATTAAAATGTGAACTATAATAAT
cDNA

GENE ATTTATAAATATATATTTATATATATTTATATATTTCTATGATTTCTTAATATATTTTC
GENE TATTTTCTTTATAAACTAAATGATTTTCACCTTCATTTTTGTATTAATAATAAACGAAC
GENE TGTATATACTAATATCAGCATGTATTTTATTTTTAACGAATAAAAGTGGACACAAGGAAA
GENE TAAATGGTAATTTCTCAATATT

489
GGGAATAAAAAAATTATAGCTCCAAGAATTTTTATTTCAGATGATAAAGACAGTTTAAAA 1800

529
GTAGAAAGAAGGGCAGAAGTAACATCAAATAATGAAGTTGTAGTTAAAGAAGAATATAAA 1920

569
GCATCATCAGCTGCTGTCGCTGTATTAGCAACTATTTTAAATGGTTTATCTTTATAAAAGA 2040

609
TCAAGAAATGATGAAATGTTAGATCCTGAGGCATCTTTTGGGGAAAGAAAAAAGAGCA 2160

622
TTCAACGTCTGATATAATCAGCTTCTCTTTTATGCTAAAAAAAAAAATATATATATATAT 2280
* END cDNA

TATGCTCATCGGATATTTCATTTTTCTTTGAGTTGATAAAATTTCCATCTTGAATTAATT 2400
TAAAAGATGTCATATACGATTTTGAACATAAAAAAAGACACACAGGAATTTAATAAAATTA 2520
AAAAATGAACAATAAAATGATTTGACATATACTATACATATGCATTAAAAAAGAGTAACTAA 2640
2662

6. A recombinant DNA molecule as claimed in claim 4 or claim 5 which is a recombinant cloning vehicle.

7. A host cell containing a recombinant DNA molecule as claimed in any one of claims 4 to 6.

8. A synthetic polypeptide prepared by expression of a nucleotide sequence as defined in claim 4 or claim 5 and displaying the antigenicity of an antigen, or an antigenic fragment thereof, as claimed in any one of claims 1 to 3.

9. A vaccine composition comprising an antigen as claimed in any one of claims 1 to 3, or an antigenic fragment thereof, or a synthetic polypeptide as claimed in claim 8, together with at least one pharmaceutically acceptable carrier or excipient.

10. A composition as claimed in claim 9 further comprising an adjuvant.

11. Use of an antigen as claimed in any one of claims 1 to 3 or a synthetic polypeptide as claimed in claim 8 for the manufacture of a vaccine composition for use in actively immunising a host against P.falciparum.

12. An antibody which is characterised by binding to an antigen as claimed in any one of claims 1 to 3.

## Patentansprüche

1. Rhoptrien-Membranantigen der asexuellen Blutstadien von Plasmodium falciparum, dadurch gekennzeichnet, daß es:
i) ursprünglich als Polypeptid mit einer relativen Molekülmasse von etwa 80000, bestimmt durch SDS-Polyacrylamid-Gelelektrophorese, synthetisiert wird;
ii) in vivo im Halsbereich der Rhoptrien von Merozoiten angeordnet ist; und
iii) Löslichkeitseigenschaften und eine hydrophobe Domäne aufweist, die typisch für ein integrales Membranprotein sind;
oder ein antigenes Fragment davon, welches zur Induktion einer schützenden Immunantwort befähigt ist.

2. Antigenfragment nach Anspruch 1, das im wesentlichen frei von anderen P.falciparum-Proteinen ist.

3. Antigen nach Anspruch 1 oder Anspruch 2, mit einer Primärstruktur, welche eine Aminosäuresequenz umfaßt, die der im folgenden angegebenen Nukleotidsequenz entspricht: 
GENE GATCTAATAACGGATATATCTATTTTTTTTAAATGCACAAGAAAAAAAAAAAAAAAGAAAAAAA
     ATATTTAATATATTTATATATATTATAAAAAAAAGGAAAAAAAAAAAAAAAAAAAAAAAAATTA

GENE GTAATATATTTTTTATTTTTAATATAAATTTTAAAAACCTAATAATTTATTTTGATATAATTTTTT

      L  S  A  F  E  F  T  Y  M  I  N  F  G  R  G  Q  N  Y  W  E
GENE TTGAGCGCCTTTGAGTTTACATATATGATATAAAACTTTGGAAGAGGACAGAATTTATTTGGGAA

      E  Y  Q  Y  P  L  H  Q  E  H  T  Y  Q  Q  E  D  S  G  E  D
GENE GAATACCAATATCCATTACACCAGGAACACATACATACCAACAAGAAGATTCAGGAGAAGAC

      E  Q  N  L  F  S  S  I  E  I  V  E  R  S  N  Y  M  G  N  P
GENE GAACAAAATTTATTTTCAAGCATTGAAAATAGTAGAAAAGAAGTAATTATATGGGTAATCCA

      D  L  G  E  D  A  E  V  A  G  T  Q  Y  R  L  P  S  G  K  C
GENE GATTTAGGAGAAGATGCTGAAGTAGCTGGAACTCAAATATAGACTTCCATCAGGGAAATGT
cDNA

      A  T  G  N  Q  Y  L  K  D  G  G  F  A  F  P  P  T  E  P  L
GENE GCTACGGGAAATCAATATTTAAAAGATGGAGGTTTTGCTTTTCCTCCAACACAGAACCTCTT
cDNA
 
AAAAAAGAACAAGGGAAAAAAAGAAGAAAAATGGAAATTAAAATTTTTACACTTATTAATAA 120
ACCATATTTAATGGTTTTATTTTTTATTTTTTTAAAAAAAAAATTGAGAATATATAAATT 240

M R K L Y C V L L 9
CAAATTAATGTACTTGTTATAAATTGTACAAAAATGAGAAAATTATACTGCGTATTATTA 360

H P Y Q K S D V Y H P I N E H R E H P K 49
CATCCATATCAAAAAAGTGATGTGTATCATCCAATCAACGAACATAGGGAACATCCAAAA 480

E N T L Q H A Y P I D H E G A E P A P Q 89
GAAAATACATTACAACACGCATATCCAATAGACCACGAAGGTGCCGAACCCGCACCACAA 600

W T E Y M A K Y D I E E V H G S G I R V 129
TGGACGGAATATATGGCAAAATATGATATTGAAGAAGTTCATGGTTCAGGTATAAGAGTA 720

P V F G K G I I I E N S N T T F L T P V 169
CCAGTATTTGGTAAAGGTATAATTATTGAGAATTCAAATACTACTTTTTTAACACCGGTA 840
cDNA START *

M S P M T L D E M R H F Y K D N K Y V K 209
ATGTCACCAATGACATTAGATGAAATGAGACATTTTTATAAAGATAATAAATATGTAAAA 960
 
N L D E L T L C S R H A G N M I P D N D
GENE AATTTAGATGAATTGACTTTATGTTCAAGACATGCAGGAAATATGATTCCAGATAATGAT
cDNA

L Y I A A Q E N N G P R Y C N K D E S K
GENE TTATATATTGCAGCTCAAGAAAATAATGGTCCTAGATATTGTAATAAAGACGAAAGTAAA
cDNA

L S K N V V D N W E K V C P R K N L Q N
GENE TTAAGTAAAAATGTAGTTGATAACTGGGAAAAAGTTTGCCCTAGAAAGAATTTACAGAAT
cDNA G (Q to

S A I D L F E C N K L V F E L S A S D Q
GENE TCAGCAATTGATCTTTTTGAATGTAATAAATTAGTTTTTGAATTGAGTGCTTCGGATCAA
cDNA A (I to N)

K N A S M I K S A F L P T G A F K A D R
GENE AAGAACGCTAGTATGATCAAAAGTGCTTTTCTTCCCACTGGTGCTTTTAAAGCAGATAGA
cDNA

E I F 'N V K P T C L I N N S Y I A T T
GENE GAAATTTTTAATGTCAAACCAACATGTTTAATTAACAATTCATCATACATTGCTACTACT
cDNA C
 
K N S N Y K Y P A V Y D D K D K K C H I 249
AAAAATTCAAATTATAAATATCCAGCTGTTTATGATGACAAAGATAAAAAGTGTCATATA 1080

R N S M F C F R P A K D I S F Q N Y T Y 289
AGAAACAGCATGTTTTGTTTTAGACCAGCAAAAGATATATCATTTCAAAACTATACATAT 1200

A K F G L W V D G N C E D I P H V N E F 329
GCAAAATTCGGATTATGGGTCGATGGAAATTGTGAAGATATACCACATGTAAATGAATTT 1320
E) G (H to D)

P K Q Y E Q H L T D Y E K I K E G F K N 369
CCTAAACAATATGAACAACATTTAACAGATTATGAAAAAATTAAAGAAGGTTTCAAAAAT 1440

Y K S H G K G Y N W G N Y N T E T Q K C 409
TATAAAAGTCATGGTAAGGGTTATAATTGGGGAAATTATAACACAGAAACACAAAAATGT 1560
T (Q to H)

A L S H P I E V E H N F P C S L Y K N E 449
GCTTTGTCCCATCCCATCGAAGTTGAACACAATTTTCCATGTTCATTATATAAAAATGAA 1680
A (H to N)
 
I M K E I E R E S K R I K L N D N D E
GENE ATAATGAAAGAAATCGAAAGAGAATCAAAACGAATTAAATTAAATGATAATGATGAA
cDNA

C P C D P E I V S N S T C N F F V C K C
GENE TGCCCATGTGACCCTGAAATTGTAAGTAATAGTACATGTAATTTCTTTGTATGTAAATGT
cDNA G (I to M) CG (N to R)

D E Y A D I P E H K P T Y D K M K I I
GENE GATGAATATGCAGATATTCCTGAACATAAACCAACTTATGATAAAATGAAAATTATAATT
cDNA

K G N A E K Y D K M D E P Q H Y G K S N
GENE AAAGGAAATGCTGAAAAATATGATAAAATGGATGAACCACAACATTATGGGAAATCAAAT
cDNA

S H T T P V L M E K P Y Y *
GENE TCACATACAACACCAGTTCTGATGGAAAAACCATACTATTAAAAATGTGAACTATAATAAT
cDNA

GENE ATTTATAAATATATTTATATATATTTATATATTTCTATGATTTCTTAATATATATTTTC
GENE TATTTTCTTTATAAACTAAAATGATTTTTCACCTTCATTTTTGTATTAATAATAAACGAAC
GENE TGTATATACTAATATATCAGCATGTATATTTTTAACGAATAAAAGTGGACACAAGGAAA
GENE TAAATGGTAATTTCTCAATATT
 
G N K K I I A P R I F I S D D K D S L K 489
GGGAATAAAAAAATTATAGCTCCAAGAATTTTTATTTCAGATGATAAAGACAGTTTAAAA 1800

V E R R A E V T S N N E V V V K E E Y K 529
GTAGAAAGAAGGGCAGAAGTAACATCAAATAATGAAGTTGTAGTTAAAGAAGAATATAAA 1920

A S S A A V A V L A T I I L M V Y L Y K R 569
GCATCATCAGCTGCTGTCGCTGTATTAGCAACTATTATTTTAATGGTTTATCTTTATAAAAGA 2040

S R N D E M L D P E A S F W G E K R A 609
TCAAGAAATGATGAAATGTTAGATCCTGAGGCATCTTTTTGGGGAGAAAAAAGAGCA 2160

622
TTCAACGTCTGATATAATCAGCTTCTCTTTTATGCTAAAAAAAAAAATATATATATATAT 2280
* END cDNA

TATGCTCATCGGATATATTTCATTTTTTCTTTGAGTTGATAAATTTCCATCTTGAATTAATT 2400
TAAAAGATGTCATATATACGATTTTGAACATAAAAAAAGACACAGGAATTTAATAAAATTA 2520
AAAAATGAACAATAAAATGATTTGACATATACTATACATATGCATTAAAAAAGAGTAACTAA 2640
2662
 oder ein antigenes Fragment davon, das zur Induktion einer schützenden Immunantwort befähigt ist.

4. Rekombinantes DNA-Molekül, umfassend eine Nukleotidsequenz, die für ein Polypeptid kodiert, das die Antigenizität eines gemäß einem der Ansprüche 1 bis 3 definierten Antigens oder eines antigenen Fragmentes davon aufweist, das zur Induktion einer schützenden Immunantwort befähigt ist.

5. Rekombinantes DNA-Molekül nach Anspruch 4, wobei die Nukleotidsequenz die folgende Sequenz vollständig oder teilweise umfaßt:

GENE GATCTAATAACGGATATATCTATTTTTTTAATGCACAAGAAAAAAAAAAAGAAAAAA
ATATTTAATATATATTTATATATAAAAAAAAAGGAAAAAAAAAAAAAAAAAAAAATTA
GENE GTAATATATATTTTTTATTTTTAATATAATTTTTAAAAACCTAATAATTTATTTTGATAATTTTT
GENE TTGAGCGCCTTTGAGTTTACATATATGATAAACTTTGGAAGAGGACAGAATTATTGGGAA
GENE GAATACCAATATCCATTACACCAGGAACATACATACCAACAAGAAGATTCAGGAGAAGAC
GENE GAACAAAAATTTATTTTCAAGCATTGAAATAGTAGAAAAGAAGTAATTATATGGGTAATCCA
GENE GATTTAGGAGAAGATGCTGAAGTAGCTGGAACTCAATATAGACTTCCATCAGGGAAATGT
cDNA
GENE GCTACGGGAAATCAATATTTAAAAGATGGAGGTTTTGCTTTTCCTCCAACAGAACCTCTT
cDNA

AAAAAAGAACAAGGGAAAAAAGAAGAAAAAATGGAAAATTAAAAATTTTTACACTTATTAATAA 120
ACCATATTTAATGGTTTTATTTTTTTATTTTTTTAAAAAAAAAAATTGAGAATATAAATT 240
9
CAAATTAATGTACTTGTTATAAATTGTACAAAAAATGAGAAAAATTATACTGCGTATTATTA 360
49
CATCCATATCAAAAAGTGATGTGTATCATCCAATCAACGAACATAGGGAACATCCAAAA 480
89
GAAAATACATTACAACACGCATATCCAATAGACCACGAAGGTGCCGAACCCGCACCACAA 600
129
TGGACGGAATATATGGCAAAATATGATATTGAAGAAGTTCATGGTTCAGGTATAAGAGTA 720
169
CCAGTATTTGGTAAAGGTATAATTATTGAGAATTCAAATACTACTTTTTAACACCGGTA 840
cDNA START *
209
ATGTCACCAATGACATTAGATGAAATGAGACATTTTTATAAAGATAATAAATATGTAAAA 960

GENE AATTTAGATGAATTGACTTTATGTTCAAGACATGCAGGAAATATGATTCCAGATAATGAT
cDNA

GENE TTATATATTGCAGCTCAAGAAAATAATGGTCCTAGATATATTGTAATAAAGACGAAAGTAAA
cDNA

GENE TTAAGTAAAAATGTAGTTGATAACTGGGAAAAAGTTTGCCCTAGAAAGAATTTACAGAAT
cDNA                                                          G (Q to

GENE TCAGCAATTGATCTTTTTGAATGTAATAAATTAGTTTTTGAATTGAGTGCTTCGGATCAA
cDNA         A (I to N)

GENE AAGAACGCTAGTATGATCAAAAGTGCTTTTCTTCCCACTGGTGCTTTTAAAGCAGATAGA
cDNA

GENE GAAATTTTAATGTCAAACCAACATGTTTAATTAACACAATTCATCATACATTGCTACT
cDNA            C

249
AAAAATTCAAATTATATAAATATCCAGCTGTTTATGATGACAAAGATAAAAAGTGTCATATA 1080

289
AGAAACAGCATGTTTTGTTTTAGACCAGCAAAAGATATATATCATTTCAAAACTATACATAT 1200

329
GCAAAATTCGGATTATGGGTCGATGGAAATTGTGAAGATATACCACATGTAAATGAATTT 1320
E)                                              G (H to D)

369
CCTAAACAACAATATGAACAACATTTAACACAGATTATGAAAAAATTAAAGAAGGTTTCAAAAAT 1440

409
TATAAAAGTCATGGTAAGGGTTATAATTGGGGAAATTATAACACACAGAAACACAAAAATGT 1560
                                                          T (Q to H)

449
GCTTTGTCCCATCCCATCGAAGTTGAACACAATTTTCCATGTTCATTATATAAAAATGAA 1680
                              A (H to N)

GENE ATAATGAAAGAAAATCGAAAGAGAATCAAAACGAATTAAATTAAATGATAATGATGATGAA
cDNA

GENE TGCCCATGTGACCCTGAAATTGTAAGTAATAGTACATGTAATTTCTTTGTATGTAAATGT
cDNA                    G (I to M)              CG (N to R)

GENE GATGAATATATGCAGATATTCCTGAACATAAACCAACTTATGATAAAATGAAAATTATAATT
cDNA

GENE AAAGGAAAATGCTGAAAAATATGATAAAATGGATGAACCACAACATTATGGGAAATCAAAT
cDNA

                                          *
GENE TCACATACAACACCAGTTCTGATGGAAAAACCATACTATTAAAATGTGAACTATAATAAT
cDNA

GENE ATTTATAAATATATTTATATATATTTATATTTCTATGATTTCTTAATATATTTTC
GENE TATTTCTTTATAAACTAAATGATTTTCACCTTCATTTTTGTATTAATAATAAACGAAC
GENE TGTATATACTAATATCAGCATGTATTTTATTTTTAACGAATAAAAGTGGACACAAGGAAA
GENE TAAATGGTAATTTCTCAATATT

GGGAATAAAAAAATTATAGCTCCAAAGAATTTTTTATTTCAGATGATAAAGACAGTTTAAAA 489
1800
GTAGAAAGAAGGCAGAAGTAACATCAAATAATGAAGTTGTAGTTAAAGAAGAATATAAA 529
1920
GCATCATCAGCTGCTGTCGCTGTATTAGCAACTATTTTAATGGTTTATCTTTATAAAAGA 569
2040
TCAAGAAAATGATGAAATGTTAGATCCTGAGGCATCTTTTGGGGAAGAAAAAGAGCA 609
2160
TTCAACGTCTGATATAATCAGCTTCTCTTTTATGCTAAAAAAAAAATATATATATAT 622
2280
• END cDNA
TATGCTCATCGGATATTTCATTTTTCTTTGAGTTGATAAAATTTCCATCTTGAATTTAATT 2400
TAAAAGATGTCATATACGATTTTGAACATAAAAAAAGACACAGGAATTTAATAAAATTA 2520
AAAAATGAACAATAAAATGATTTGACATACTATACATATGCATTAAAAAAGAGTAACTAA 2640
2662

6. Rekombinantes DNA-Molekül nach Anspruch 4 oder Anspruch 5, das als rekombinantes Klonierungs-Vehikel vorliegt.

7. Wirtszelle, enthaltend ein rekombinantes DNA-Molekül nach einem der Ansprüche 4 bis 6.

8. Synthetisches Peptid, hergestellt durch Expression einer Nukleotidsequenz gemäß Anspruch 4 oder Anspruch 5, das die Antigenizität eines Antigens oder eines antigenen Fragmentes gemäß einem der Ansprüche 1 bis 3 aufweist.

9. Vakzine-Zusammensetzung, umfassend ein Antigen gemäß einem der Ansprüche 1 bis 3 oder ein antigenes Fragment davon, oder ein synthetisches Polypeptid gemäß Anspruch 8, zusammen mit wenigstens einem pharmazeutisch akzeptablen Träger oder Exzipienten.

10. Zusammensetzung nach Anspruch 9, die außerdem ein Adjuvans umfaßt.

11. Verwendung eines Antigens nach einem der Ansprüche 1 bis 3 oder eines synthetischen Polypeptids nach Anspruch 8 zur Herstellung einer Vakzinezusammensetzung zur Verwendung bei der aktiven Immunisierung eines Wirts gegen P.falciparum.

12. Antikörper, gekennzeichnet durch die Bindung an ein Antigen gemäß einem der Ansprüche 1 bis 3.

## Revendications

1. Antigène de membrane de rhoptry des phases sanguines asexuées de Plasmodium falciparum, qui se caractérise par le fait :
i) d'être initialement synthétisé sous forme de polypeptide possédant une masse moléculaire relative d'approximativement 80.000, comme déterminé par électrophorèse sur gel de polyacrylamide.DSS,
ii) d'être situé in vivo dans la région étranglée des rhoptrys de rnérozoïtes et
iii) de posséder les caractéristiques de solubilité et un domaine hydrophobe typique d'une protéine de membrane intégrale,
ou un fragment antigénique de celui-ci, capable d'induire une immunoréponse protectrice.

2. Fragment d'antigène suivant la revendication 1, caractérisé en ce qu'il est sensiblement exempt d'autres protéines de P. falciparum.

3. Antigène suivant la revendication 1 ou la revendication 2 possédant une structure primaire qui comprend la séquence d'aminoacides correspondant à la séquence de nucléotides indiquée : 
GENE GATCTAATAACGGATATATCTATTTTTTTAATGCACAAGAAAAAAAAAAAAAAGAAAAAA
ATATTTAATATATTTATATATTATATAAAAAAAAAGGAAAAAAAAAAAAAAAAAAAAATTA

GENE GTAATATATATTTTTATTTTAATATATAATTTTAAAAACCTAATAATAATTTATTTGATAATTTTTT

L S A F E F T Y M I N F G R G Q N Y W E
GENE TTGAGCGCCTTTGAGTTTACATATATGATAAACTTTGGAAGAGGACAGAATTATTGGGAA

E Y Q Y P L H Q E H T Y Q Q E D S G E D
GENE GAATACCAATATCCATTACACCAGGAACATACATACCAACAAGAAGATTCAGGAGAAGAC

E Q N L F S S I E I V E R S N Y M G N P
GENE GAACAAAATTTATTTTCAAGCATTGAAATAGTAGAAAGAAGTAATTATATGGGTAATCCA

D L G E D A E V A G T Q Y R L P S G K C
GENE GATTTAGGAGAAGATGCTGAAGTAGCTGGAACTCAATATAGACTTCCATCAGGGAAATGT
cDNA

A T G N Q Y L K D G G F A F P P T E P L
GENE GCTACGGGAAATCAATATTTAAAAGATGGAGGTTTTGCTTTTCCTCCAACAGAACCTCTT
cDNA
 
AAAAAAGAACAAGGGAAAAAAGAAGAAAAATGGAAATTAAAATTTTACACTTATTAATAA 120
ACCATATTTAATGGTTTTATTTTTTATTTTTTTAAAAAAAAAAATTGAGAATATAAATT 240

M R K L Y C V L L 9
CAAATTAATGTACTTGTTATAAATTGTACAAAAATGAGAAAATTATACTGCGTATTATTA 360

H P Y Q K S D V Y H P I N E H R E H P K 49
CATCCATATCAAAAAAGTGATGTGTATCATCCAATCAACGAACATAGGGAACATCCAAAA 480

E N T L Q H A Y P I D H E G A E P A P Q 89
GAAAATACATTACAACACGCATATCCAATAGACCACGAAGGTGCCGAACCCGCACCACAA 600

W T E Y M A K Y D I E E V H G S G I R V 129
TGGACGGAATATATGGCAAAATATGATATTGAAGAAGTTCATGGTTCAGGTATAAGAGTA 720

P V F G K G I I I E N S N T T F L T P V 169
CCAGTATTTGGTAAAGGTATAATTATTGAGAATTCAAATACTACTTTTTTAACACCGGTA 840
cDNA START *

M S P M T L D E M R H F Y K D N K Y V K 209
ATGTCACCAATGACATTAGATGAAATGAGACATTTTTATAAAGATAATAAATATGTAAAA 960
 
N L D E L T L C S R H A G N M I P D N D
GENE AATTTAGATGAATTGACTTTATGTTCAAGACATGCAGGAAATATGATTCCAGATAATGAT
cDNA

L Y I A A Q E N N G P R Y C N K D E S K
GENE TTATATATTGCAGCTCAAGAAAATAATGGTCCTAGATATTGTAATAAAGACGAAAGTAAA
cDNA

L S K N V V D N W E K V C P R K N L Q N
GENE TTAAGTAAAAATGTAGTTGATAACTGGGAAAAAGTTTGCCCTAGAAAGAATTTACAGAAT
cDNA G (Q to

S A I D L F E C N K L V F E L S A S D Q
GENE TCAGCAATTGATCTTTTTGAATGTAATAAATTAGTTTTTGAATTGAGTGCTTCGGATCAA
cDNA A (I to N)

K N A S M I K S A F L P T G A F K A D R
GENE AAGAACGCTAGTATGATCAAAAGTGCTTTTCTTCCCACTGGTGCTTTTAAAGCAGATAGA
cDNA

E I F 'N V K P T C L L I N N S Y I A T T
GENE GAAATTTTTAATGTCAAACCAACATGTTTATTAATTAACAATTCATCATACATTGCTACTACT
cDNA C
 
K N S N Y K Y P A V Y Y D D K D K K C H I 249
AAAAATTCAAATTATAAATATCCAGCTGTTTATTATGATGACAAAGATAAAAAGTGTCATATA 1080

R N S M F C F R P A K D I S F Q N Y T Y 289
AGAAACAGCATGTTTTGTTTTAGACCAGCAAAAGATATATCATTTCAAAACTATACATAT 1200

A K F G L W V D G N C E D I P H V N E F 329
GCAAAATTCGGATTATGGGTCGATGGAAATTGTGAAGATATACCACATGTAAATGAATTT 1320
 E)                                            G (H to D)

P K Q Y E Q H L T D Y E K I K E G F K N 369
CCTAAACAATATGAACAACATTTAACAGATTATGAAAAAATTAAAGAAGGTTTCAAAAAT 1440

Y K S H G K G Y N W G N Y N T E T Q K C 409
TATAAAAGTCATGGTAAGGGTTATAATTGGGGAAATTATAACACAGAAACACAAAAATGT 1560
                                                      T (Q to H)

A L S H P I E V E H N F P C S L Y K N E 449
GCTTTGTCCCATCCCATCGAAGTTGAACACAATTTTCCATGTTCATTATATAAAAATGAA 1680
                              A (H to N)
 
I M K E I E R E S K R I K L N D N D D E
GENE ATAATGAAAGAAATCGAAAGAGAATCAAAACGAATTAAATTAAATGATAATGATGATGAA
cDNA

C P C D P E I V S N S T C N F F V C K C
GENE TGCCCATGTGACCCTGAAATTGTAAGTAATAGTACATGTAATTTCTTTGTATGTAAATGT
cDNA G (I to M) CG (N to R)

D E Y A D I P E H K P T Y D K M K I I I
GENE GATGAATATGCAGATATTCCTGAACATAAACCAACTTATGATAAAATGAAAATTATAATT
cDNA

K G N A E K Y D K M D E P Q H Y G K S N
GENE AAAGGAAATGCTGAAAAATATGATAAAATGGATGAACCACAACATTATGGAAAATCAAAT
cDNA

S H T T P V L M E K P Y Y *
GENE TCACATACAACACCAGTTCTGATGGAAAAACCATACTATTAAAAATGTGAACTATAATAAT
cDNA

GENE ATTTATAAATATATTTATATATATTTATATATTTCTATGATTTCTTAATATATTTTC
GENE TATTTTCTTTATAAACTAAATGATTTTCACCTTCATTTTTGTATTAATAATAAACGAAC
GENE TGTATATACTAATATATCAGCATGTATTTTATTTTTAACGAATAAAAGTGGACACAAGGAAA
GENE TAAATGGTAATTTCTCAATATATT
 
G N K K I I A P R I F I S D D K D S L K 489
GGGAATAAAAAAATTATAGCTCCAAGAATTTTTATTTCAGATGATAAAGACAGTTTAAAA 1800

V E R R A E V T S N N E V V V K E E Y K 529
GTAGAAAGAAGGGCAGAAGTAACATCAAATAATGAAGTTGTAGTTAAAGAAGAATATAAA 1920

A S S A A V A V L A T I L M V Y L Y K R 569
GCATCATCAGCTGCTGTCGCTGTATTAGCAACTATTTTAATGGTTTATCTTTATAAAAGA 2040

S R N D E M L D P E A S F W G E E K R A 609
TCAAGAAATGATGAAATGTTAGATCCTGAGGCATCTTTTTGGGGAGAAGAAAAAAGAGCA 2160

622
TTCAACGTCTGATATAATCAGCTTCTCTTTTATGCTAAAAAAAAATATATATATATATAT 2280
* END cDNA

TATGCTCATCGGATATTTCATTTTTCTTTGAGTTGATAAATTTCCATCTTGAATTTAATT 2400
TAAAAGATGTCATATACGATTTTGAACATAAAAAAAGACACAGGAATTTAATAAAAATTA 2520
AAAAATGAACAATAAAAATGATTTGACATACTATACATATGCATTAAAAAGAGTAACTAA 2640
2662
 ou un fragment antigénique de celui-ci capable, d'induire une immunoréponse protectrice.

4. Molécule d'ADN recombinant comprenant une séquence de nucléotides encodant un polypeptide possédant l'antigénicité d'un antigène suivant l'une quelconque des revendications 1 à 3, ou d'un fragment antigénique de celui-ci, capable d'induire une immunoréponse protectrice.

5. Molécule d'ADN recombinant suivant la revendication 4, caractérisée en ce que ladite séquence de nucléotides comprend la totalité ou une partie de la séquence suivante :

GENE GATCTAATAACGGATATATCTATTTTTTTAATGCACAAGAAAAAAAAAAAAAAGAAAAAA
ATATTTAATATATTTATATATTATATAAAAAAAAAAGGAAAAAAAAAAAAAAAAAAAATTA

GENE GTAATATATATTTTTTATTTTTAATATATAATTTTTAAAAACCTAATAATAATTTATTTTGATAATTTTTT

GENE TTGAGGCGCCTTTGAGTTTACATATATGATAAACTTTGGAAGAGGACAGAATTATTGGGAA

GENE GAATACCAATATCCATTACACCAGGAACATACATACCAACAAGAAGATTCAGGAGAAGAC

GENE GAACAAAATTTATTTTCAAGCATTGAAATAGTAGAAAAGAAAGTAATTATATGGGTAATCCA

GENE GATTTAGGAGAAGATGCTGAAGTAGCTGGAACTCAATATAGACTTCCATCAGGGAAAATGT
cDNA

GENE GCTACGGGAAATCAATATATTTAAAAGATGGAGGTTTTGCTTTTCCTCCAACAGAACCTCTT
cDNA

AAAAAAGAACAAGGGAAAAAAGAAGAAAAAATGGAAAATTAAAAATTTTTACACTTATTAATAA 120
ACCATATTTAATGGTTTTATTTTTTTATTTTTTTAAAAAAAAAAATTGAGAAATATAAATT 240
9
CAAATTAATGTACTTGTTATAAATTGTACAAAAATGAGAAAAATTATACTGCGTATTATTA 360
49
CATCCATATCAAAAAAGTGATGTGTATCATCCAATCAACGAACATAGGGAACATCCAAAA 480
89
GAAAATACATTACAACACGCATATCCAATAGACCACGAAGGTGCCGAACCCGCACCACAA 600
129
TGGACGGAATATATGGCAAAATATGATATTGAAGAAGTTCATGGTTCAGGTATAAGAGTA 720
169
CCAGTATTTGGTAAAGGTATAATTATTGAGAATTCAAATACTACTTTTTAACACCGGTA 840
cDNA START *
209
ATGTCACCAATGACATTAGATGAAATGAGACATTTTTATAAAGATAAATATGTAAAA 960

GENE AATTTAGATGAATTGACTTTATGTTCAAGACATGCAGGAAAATATGATTCCAGATAATGAT
cDNA

GENE TTATATATTGCAGCTCAAGAAAAATAAATGGTCCTAGATATTGTAATAAAAGACGAAAGTAAA
cDNA

GENE TTAAGTAAAAATGTAGTTGATAACTGGGAAAAAGTTTGCCCTAGAAAGAATTTACAGAAT
cDNA                                                        G (Q to

GENE TCAGCAATTGATCTTTTTGAATGTAATAAATTAGTTTTTGAATTGAGTGCTTCGGATCAA
cDNA          A (I to N)

GENE AAGAACGCTAGTATGATCAAAAGTGCTTTTCTTCCCACTGGTGCTTTTAAAGCAGATAGA
cDNA

GENE GAAATTTTTAATGTCAAACCAACATGTTTAATTAACAATTCATCATACATTGCTACTACT
cDNA         C

                                                                    249
AAAAATTCAAATTATAAATATCCAGCTGTTTATGATGACAAAGATAAAAAGTGTCATATA 1080

                                                                    289
AGAAACAGCATGTTTTGTTTTAGACCAGCAAAAAGATATATCATTTCAAAACTATACATAT 1200

                                                                    329
GCAAAATTCGGATTATGGGTCGATGGAAATTGTGAAGATATACCACATGTAAATGAATTT 1320
 E)                                          G (H to D)

                                                                    369
CCTAAACAATATGAACAACATTTAACAGATTATGAAAAAATTAAAGAAGGTTTCAAAAAT 1440

                                                                    409
TATAAAAGTCATGGTAAGGGTTATAATTGGGGAAAATTATAACACACAGAAACACAAAATGT 1560
                                                          T (Q to H)

                                                                    449
GCTTTGTCCCATCCCATCGAAGTTGAACACAATTTTCCATGTTCATTATATAAAAATGAA 1680
                              A (H to N)

GENE ATAATGAAAAGAAATCGAAAGAGAGAATCAAAACGAATTAAATTAAATGATAATGATGATGAA
cDNA

GENE TGCCCATGTGACCCTGAAATTGTAAGTAATAGTACATGTAATTTCTTGTATGTAAATGT
cDNA                        G (I to M)              CG (N to R)

GENE GATGAATATATGCAGATATTCCTGAACATAAACCAACTTATGATAAAAATGAAAAATIATAATT
cDNA

GENE AAAGGAAAATGCTGAAAAAATATGATAAAATGGATGAACCACAACATTATGGAAAATCAAAT
cDNA
                                                  *
GENE TCACATACAACACCAGTTCTGATGGAAAAACCATACTATTAAAATGTGAACTATAATAAT
cDNA

GENE ATTTATAAATATATTTATATATATTTATATATTTATATTTCTATGATTTCTTAATATATTTTC
GENE TATTTTCTTTATAAACTAAATGATTTTCACCTTCATTTTTGTATTAATAATAAACGAAC
GENE TGTATATACTAATATATCAGCATGTATATTTTATTTTTAACGAATATAAAAGTGGACACAAGGAAA
GENE TAAATGGTAATTTCTCAATATATT

489
GGGAATAAAAAAATTATAGCTCCAAGAATTTTTATTTCAGATGATAAAGACAGTTTAAAA 1800

529
GTAGAAAGAAGGGCAGAAGTAACATCAAATAATGAAGTTGTAGTTAAAGAAGAATATAAA 1920

569
GCATCATCAGCTGCTGTCGCTGTATTTAGCAACTATTTTAATGGTTTATCTTTATAAAGA 2040

609
TCAAGAAATGATGAAATGTTAGATCCTGAGGCATCTTTTGGGGAAGAAAAAAGAGCA 2160

622
TTCAACGTCTGATATATAATCAGCTTCTCTCTTTTATGCTAAAAAAAAAAAATATATATATAT 2280
* END cDNA

TATGCTCATCGGATATTTCATTTTTCTTTGAGTTGATAAAATTTCCATCTTGAATTAATT 2400
TAAAAGATGTCATATACGATTTTGAACATAAAAAAAGACACAGGAATTTAATAAAATTA 2520
AAAAATGAACAATAAAATGATTTGACATATACTATACATATGCATTAAAAAAGAGTAACTAA 2640
2662

6. Molécule d'ADN recombinant suivant la revendication 4 ou la revendication 5, qui est un véhicule clonant recombinant.

7. Cellule hôte contenant une molécule d'ADN recombinant suivant l'une quelconque des revendications 4 à 6.

8. Polypeptide synthétique, préparé par l'expression d'une séquence de nucléotides telle que définie dans la revendication 4 ou la revendication 5 et présentant l'antigénicité d'un antigène ou d'un fragment antigénique de celui-ci, suivant l'une quelconque des revendications 1 à 3.

9. Composition de vaccin comprenant un antigène suivant l'une quelconque des revendications 1 à 3, ou un fragment antigénique de celui-ci, ou un polypeptide synthétique suivant la revendication 8, ainsi qu'au moins un excipient ou véhicule pharmaceutiquement acceptable.

10. Composition suivant la revendication 9, caractérisée, en ce qu'elle comprend aussi un adjuvant.

11. Utilisation d'un antigène suivant l'une quelconque des revendications 1 à 3, ou d'un polypeptide synthétique suivant la revendication 8, pour la fabrication d'une composition de vaccin à utiliser pour activement immuniser un hôte contre P. falciparum.

12. Anticorps qui est caractérisé par le fait de se lier à un antigène suivant l'une quelconque des revendications 1 à 3.
